# EUROPEAN PATENT APPLICATION

(11) **EP 4 417 709 A1**
(43) Date of publication of application: **21.08.2024**
(21) Application number: 23156564.9
(22) Date of filing: 14.02.2023
(51) Int. Cl.: C12Q 1/6886

(54) **MIRNA-BASED DIAGNOSTIC ASSAY FOR THE DETECTION OF A RISK TO DEVELOP SKIN TUMORS AGAINST THE BACKGROUND OF SEVERE SKIN LESIONS**

(71) Applicant: Gemeinnützige Salzburger Landeskliniken Betriebsges.mbH, 5020 Salzburg (AT)
(72) Inventor: Wally, Verena, A-5020 Salzburg (AT); Wimmer, Monika, A-5020 Salzburg (AT); Zauner, Roland, A-5020 Salzburg (AT); Ablinger, Michael, A-5020 Salzburg (AT)
(74) Representative: Krauss, Jan

(57) **Abstract**

The present invention relates to a method for diagnosing the risk of a subject to suffer from squamous cell carcinoma (SCC), comprising detecting the expression level of the miR-187-3p molecule, in a sample obtained from said subject. In another preferred aspect, the method according to the present invention further comprises detecting the expression level of at least one of the miR-27b-3p, miR-301a-3p, or miR-1260a, in said sample obtained from said subject. A changed expression level of said at least one miRNA, as compared to healthy donors, further indicates an increased risk of said subject to suffer from squamous cell carcinoma. The subject to be diagnosed may suffer from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB). Other aspects relate to kits and diagnostic compositions, as well as to an improved treatment of SCC, in particular in a background of RDEB.

## Description

The present invention relates to a method for diagnosing the risk of a subject to suffer from squamous cell carcinoma (SCC), comprising detecting the expression level of the miR-187-3p molecule, in a sample obtained from said subject. In another preferred aspect, the method according to the present invention further comprises detecting the expression level of at least one of the miR-27b-3p, miR-301a-3p, or miR-1260a, in said sample obtained from said subject. A changed expression level of said at least one miRNA, as compared to healthy donors, further indicates an increased risk of said subject to suffer from squamous cell carcinoma. The subject to be diagnosed may suffer from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB). Other aspects relate to kits and diagnostic compositions, as well as to an improved treatment of SCC, in particular in a background of RDEB.

### Background of the invention

The disruption of the epidermal barrier, presenting as blisters and wounds, is the defining characteristic of the severe recessive subtype of dystrophic epidermolysis bullosa (RDEB), a genetic skin disease caused by mutations within the COL7A1 gene, resulting in dysfunctional or loss of the structural extracellular matrix protein collagen type VII (C7) and accounting for 11% of all EB cases (Has et al. 2020, Kim et al. 2018). While the majority of blisters heal within a few weeks, about one in four causes more severe problems due to their cyclic recurrence or by evolving into long-standing chronic open wounds (Solis et al. 2021).

Mechanistically, impaired wound healing is considered to be a consequence of the lack of C7, which in turn leads to an altered migratory behavior of fibroblasts and keratinocytes, matrix remodeling, accumulation of mutations, bacterial challenge and an overall, excessive inflammatory milieu (Woodley et al. 2021, Wimmer et al. 2020, Nyström et al. 2013, Nyström et al. 2015, Cho et al. 2018, Nyström et al. 2018, Phillips et al. 2020, Tartaglia G, Cao Q, Padron ZM, South AP. Impaired Wound Healing, Fibrosis, and Cancer: The Paradigm of Recessive Dystrophic Epidermolysis Bullosa. Int J Mol Sci. 2021 May 12;22(10):5104. doi: 10.3390/ijms22105104. PMID: 34065916; PMCID: PMC8151646). In addition, it is likely that factors such as loss of microbiome diversity, stem cell depletion, recurrent trauma and impaired cell proliferation contribute to a compromised wound healing in RDEB (Fuentes et al. 2018, Solis et al. 2021, Cianfarani et al. 2017).

Not only do chronic wounds burden patients with significant pain and reduced life quality, they are also associated with a life-threatening complication in RDEB - the development of aggressive squamous cell carcinomas (SCCs) (Solis et al. 2021, Fine et al. 2009). Indeed, chronic, non-healing wounds are the primary (> 80%) sites for the emergence of SCCs. The cumulative risk of RDEB patients to develop an SCC by the age of 35 is approximately 68%, and attaining 90% by 55 years according to the U.S. national EB registry (NEBR). In contrast to non-EB SCCs, tumors arising in individuals with RDEB are particularly aggressive, as they show a high recurrence rate and early metastatic spread, accounting for a mortality rate of 87 % by the age of 45 upon SCC development (Fine et al. 2009).

Thus, early diagnosis is of highest prognostic relevance.

However, assessment only on clinical grounds is often infeasible in the background of chronic wounding, granulation, crusting and scarring. Currently, detection of RDEB-SCCs requires meticulous surveillance of non-healing wounds and necessitates invasive skin biopsies to confirm malignant lesions, which pose a major burden to patients. Moreover, full bioptic coverage of suspicious areas is often infeasible. (Condorelli et al. 2019, Mellerio et al. 2016).

US20120059048A1 describes diagnostic methods relating to detecting miRNA-205, thereby diagnosing an aggressive form of squamous cell carcinoma.

To date, there is very limited data on diagnostic procedures which are less invasive and that can be used to diagnose SCCs. One report suggests the use of dermatoscopy, which requires to be conducted by experienced, specially trained dermatologists (Toncic et al. 2018).

Another study described the potential of exploiting an RDEB-SCC specific exosomal marker, however, currently demonstrated only in a xenograft mouse model (Sun et al. 2018). Recently, tumor-derived serine proteases C1r and C1s, which are part of the classical pathway of the complement system, have been proposed as biomarkers of aggressive SCCs, including RDEB-SCCs (Riihillä et al. 2019). However, they were proposed as markers of tumor progression and not analyzed for their potential in early detection, probably due to the fact that only slight differences between normal skin, actinic keratosis and SCC in situ (i.e., early-stage tumors) were observed. In addition, no comparison with wound tissue was performed.

Although not specifically addressing a potential use as tumor marker, Chacon et al. proposed periostin as a serum marker for RDEB to monitor treatment success of anti-fibrotic therapies (Chacón-Solano et al. 2008), whereas MMP-7 was characteristically expressed at the invasive edge of RDEB-SCCs and was thus discussed as a therapeutic target or biomarker for SCC progression. (Kivisaari et al. 2008). The same group also found MMP-13 to be highly expressed by RDEB-SCCs and also SerpinA1 expression was shown to correlate with SCC progression (Kivisaari et al. 2008, Farshchian et al. 2011).

In another study, RDEB-SCCs but not non-malignant keratinocytes were found to specifically express the cancer-type transcript of the solute carrier organic anion transporter family member 1B3 protein (Ct-SLCO 1B3). Its potential utility as a detection marker was proposed, as its RNA could be detected in extracellular vesicles (EVs) harvested from conditioned medium of RDEB-SCC cells cultured in vitro, and from serum of tumor-bearing mice, but not in respective control samples. (Sun et al. 2018, Gruber et al. 2013).

None of these markers were further evaluated in a clinical setting and none has been investigated for its utility in early detection, as well as its accessibility using non-invasive methods of sampling.

Vanden Oever et al. (in: miR-29 Regulates Type VII Collagen in Recessive Dystrophic Epidermolysis Bullosa. J Invest Dermatol. 2016 Oct;136(10):2013-2021. doi: 10.1016/j.jid.2016.05.115. Epub 2016 Jun 18. PMID: 27328306; PMCID: PMC5341382) describe a mechanism of regulation, to our knowledge previously unknown, by which micro RNA-29 (miR-29) regulates COL7A1 in a complex network: both directly through targeting its 3' untranslated region at two distinct seed regions and indirectly through targeting an essential transcription factor required for basal COL7A1 expression, SP1. In turn, miR-29 itself is regulated by SP1 activity and transforming growth factor-β signaling. RDEB mice express high levels of transforming growth factor-β and significantly lower miR-29 compared with wildtype cohorts.

Condorelli et al. (in: MicroRNA-145-5p regulates fibrotic features of recessive dystrophic epidermolysis bullosa skin fibroblasts. Br J Dermatol. 2019 Nov;181(5):1017-1027. doi: 10.1111/bjd.17840. Epub 2019 Oct 20. PMID: 30816994) describe that the miR-143/145 cluster was upregulated in RDEBFs compared with fibroblasts from healthy subjects. Their results highlight the profibrotic role of miR-145-5p and its regulatory networks in RDEB.

EP2794881B1 describes miRNA for treating head and neck cancer and relates to the diagnostic and therapeutic uses of a miRNA-323, miRNA-342, miRNA-326, miRNA-371, miRNA-3157 and/or miRNA-345 molecule in a disease and condition associated with a squamous cell carcinoma such as head and neck cancer or a preneoplastic mucosal change.

Wimmer et al. (in: A cancer stem cell-like phenotype is associated with miR-10b expression in aggressive squamous cell carcinomas. Cell Commun Signal. 2020 Apr 10;18(1):61. doi: 10.1186/s12964-020-00550-9. PMID: 32276641; PMCID: PMC7146875) disclose that cutaneous squamous cell carcinomas (cSCC) are the primary cause of premature deaths in patients suffering from the rare skin-fragility disorder recessive dystrophic epidermolysis bullosa (RDEB), which is in marked contrast to the rarely metastasizing nature of these carcinomas in the general population. MiRNA expression profiling was performed across various cell types isolated from skin and cSCCs. Several miRNAs were identified as dysregulated in cSCCs compared to control skin. The metastasis-linked miR-10b, which was significantly upregulated in primary cell cultures and in archival biopsies. Analysis ofmiR-10b downstream effects identified a novel putative target of miR-10b, the actin- and tubulin cytoskeleton-associated protein DIAPH2. This is said to provide an important avenue for future development of novel therapies targeting this metastasis-linked miRNA. The miRNAs as identified do not overlap with the ones of the present invention.

It is therefore an object of the present invention to provide diagnostic markers that allow for an early detection and/or prognosis of the development of tumors and cancer in patients suffering from wound healing disorders, such as found in RDEB and related diseases. A further object of the present invention is the development of non-invasive tests that allow for a regular testing in the patients without invasive sample collection and thus high compliance.

The problem of the present invention is solved by providing a method for diagnosing the risk of a subject to suffer from squamous cell carcinoma (SCC), comprising detecting the expression level of the miR-187-3p molecule, an isomiR, or a precursor thereof in a sample obtained from said subject, wherein a changed expression level of said miRNA, as compared to healthy donors, indicates an increased risk of said subject to suffer from squamous cell carcinoma.

Preferred is the method according the present invention, further comprising detecting the expression level of at least one of the miR-27b-3p, miR-301a-3p, or miR-1260a, an isomiR, or a precursor thereof in said sample obtained from said subject, wherein a changed expression level of said at least one miRNA, as compared to healthy donors, further indicates an increased risk of said subject to suffer from squamous cell carcinoma.

Further preferred is the method according the present invention, wherein a decrease of the expression level of miR-301a-3p and/or miR-1260a when compared to healthy donors indicates an increased risk of said subject to suffer from squamous cell carcinoma, and wherein an increase of the expression level of miR-187-3p and/or miR-27b-3p when compared to healthy donors indicates an increased risk of said subject to suffer from squamous cell carcinoma.

Optimally, the sample as used in the methods according to the present invention comprises a liquid biopsy and/or swap sample, such as a wound swab sample, from said subject. The subject may suffer from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

The problem of the present invention is further solved by providing a diagnostic composition comprising a sample obtained from a subject at risk to suffer from squamous cell carcinoma (SCC) comprising miR-187-3p molecule, an isomiR, or a precursor thereof, preferably further comprising at least one of miR-27b-3p, miR-301a-3p, or miR-1260a molecule, an isomiR, or a precursor thereof.

Preferred is the diagnostic composition according to the present invention, wherein the sample comprises a liquid biopsy and/or swap sample, such as a wound swab sample, from said subject. Further preferred is the diagnostic composition according to the present invention, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

The problem of the present invention is further solved by providing a kit for in vitro assessment of the risk that a subject suffers from squamous cell carcinoma (SCC), comprising buffers and reagents to perform a method according to the present invention, optionally together with instructions for performing said method. The present invention also encompasses the use of the kit according to the present invention for diagnosing a subject to suffer from SCC, in particular cutaneous squamous cell carcinoma (cSCC), in particular according to a method according to the present invention.

The problem of the present invention is also solved by providing a computer program for identifying a subject's risk to suffer from SCC, in particular cutaneous squamous cell carcinoma (cSCC), in particular as determined according to a method according to the present invention, wherein said program is preferably implemented to run on a computer.

The problem of the present invention is also solved by providing a method for preventing or treating squamous cell carcinoma (SCC), in particular cSCC, in a subject, comprising the steps of performing method according to to the present invention, to determine if the subject has an increased probability of suffering or is suffering from squamous cell carcinoma (SCC), in particular cSCC, and selecting an appropriate prevention or therapy for the subject based on the determination.

As mentioned above, in a first aspect of the present invention, the object of the present invention is solved by a method for diagnosing the risk of a subject to suffer from squamous cell carcinoma (SCC), comprising detecting the expression level of the miR-187-3p molecule, an isomiR, or a precursor thereof in a sample obtained from said subject, wherein a changed expression level of said miRNA, as compared to healthy donors, indicates an increased risk of said subject to suffer from squamous cell carcinoma. In another preferred aspect, the method according to the present invention further comprises detecting the expression level of at least one of the miR-27b-3p, miR-301a-3p, or miR-1260a, an isomiR, or a precursor thereof in said sample obtained from said subject, wherein a changed expression level of said at least one miRNA, as compared to healthy donors, further indicates an increased risk of said subject to suffer from squamous cell carcinoma.

Several micro-RNAs (miRNA) play an important role in the generation and spread of cancer and tumors. The present inventors were able to obtain miRNAs from tumor swabs, and to characterize those that allow to identify tumorous tissue. An early diagnosis of the tumors that are otherwise very difficult to detect is important in order to still allow for an effective surgical removal.

Currently, there are no non-invasive methods for the early detection of squamous cell carcinoma in EB. Currently, the diagnosis of squamous cell carcinomas in patients with epidermolysis bullosa is carried out visually and in case of suspected malignancy by punch biopsies, which represent a massive burden for patients and also involve the risk of overlooking malignant lesions, as these are very difficult to detect in large, chronic wounds. In the context of epidermolysis bullosa, there are publications describing the role of certain miRNAs in EB.

miR-187-3p, a recently identified cancer-related microRNA, was previously reported to play promoting or suppressive roles in different malignancies, such as HCC. miR-187-3p is significantly down-regulated in HCC, and is correlated with adverse clinical features and poor prognosis of HCC patients. (Dou C, Liu Z, Xu M, Jia Y, Wang Y, Li Q, Yang W, Zheng X, Tu K, Liu Q. miR-187-3p inhibits the metastasis and epithelial-mesenchymal transition of hepatocellular carcinoma by targeting S100A4. Cancer Lett. 2016 Oct 28;381(2):380-90. doi: 10.1016/j.canlet.2016.08.011. Epub 2016 Aug 17. PMID: 27544906).

Sreedharan L, et al. (in: MicroRNA profile in neosquamous esophageal mucosa following ablation of Barrett's esophagus. World J Gastroenterol. 2017 Aug 14;23(30):5508-5518. doi: 10.3748/wjg.v23.i30.5508. PMID: 28852310; PMCID: PMC5558114) describe nine microRNAs (miR-424-5p, miR-127-3p, miR-98-5p, miR-187-3p, miR-495-3p, miR-34c-5p, miR-223-5p, miR-539-5p, miR-376a-3p, miR-409-3p) that were expressed at higher levels in post-ablation neosquamous mucosa than in matched proximal squamous and healthy squamous mucosa. These microRNAs were also more highly expressed in Barrett's esophagus mucosa than matched proximal squamous and squamous mucosa from controls.

Han M, et al. (in: MiR-27b-3p exerts tumor suppressor effects in esophageal squamous cell carcinoma by targeting Nrf2. Hum Cell. 2020 Jul;33(3):641-651. doi: 10.1007/s13577-020-00329-7. Epub 2020 May 17. PMID: 32419118) disclose that miR-27b-3p has been reported to function as tumor suppressor in several tumors, including breast cancer and lung cancer. Also, miR-27b-3p has been identified to be significantly down-regulated in esophageal cancer.

Zhang N, and Liu JF (in: MicroRNA (MiR)-301a-3p regulates the proliferation of esophageal squamous cells via targeting PTEN. Bioengineered. 2020 Dec; 11(1):972-983. doi: 10.1080/21655979.2020.1814658. PMID: 32970954; PMCID: PMC8291791) explore the mechanism of miR-301a-3p regulating the proliferation of esophageal squamous cell carcinoma (ESCC) cells. Compared with adjacent normal esophageal tissues, the relative level of miR-301a-3p/U6 was elevated in ESCC tissues. MiR-301a-3p could facilitate ESCC cell proliferation. And miR-301a-3p directly bind to PTEN 3'-UTR and negatively regulated PTEN protein expression.

The RNA-sequences of the most preferred miRNA molecules as used in the invention are:
miR-187-3p: UCGUGUCUUGUGUUGCAGCCGG (SEQ ID NO: 1);
miR-27b-3p: UUCACAGUGGCUAAGUUCUGC (SEQ ID NO:2);
miR-301a-3p: CAGUGCAAUAGUAUUGUCAAAGC (SEQ ID NO: 3); and
miR-1260a: AUCCCACCUCUGCCACCA (SEQ ID NO: 4).

In the present context the term "miRNA" shall mean to include a miRNA molecule, a miRNA equivalent, a miRNA mimic or a miRNA isomiR, or a mimic or a suitable isomiR or a miRNA source or a precursor thereof. A preferred equivalent is an isomiR or a mimic. In the context of the invention, a miRNA molecule or an equivalent or a mimic or an isomiR thereof may be a synthetic or natural or recombinant or mature or part of a mature miRNA or a human miRNA or derived from a human miRNA as further defined in the part dedicated to the general definitions. A human miRNA molecule is a miRNA molecule which is found in a human cell, tissue, organ or body fluids (i.e. endogenous human miRNA molecule). A human miRNA molecule may also be a human miRNA molecule derived from an endogenous human miRNA molecule by substitution, deletion and/or addition of a nucleotide. A miRNA molecule or an equivalent or a mimic thereof may be a single stranded or double stranded RNA molecule. Preferably a miRNA molecule or an equivalent, or a mimic thereof is from 6 to 30 nucleotides in length, preferably 12 to 30 nucleotides in length, preferably 15 to 28 nucleotides in length, more preferably said molecule has a length of at least 6, 7, 8, 9, 10, 11, 12, 13, 14, 15, 16, 17, 18, 19, 20, 21, 22, 23, 24, 25, 26, 27, 28, 29, 30 nucleotides or more. Preferred is a miRNA molecule or an equivalent or a mimic or isomiR thereof that is from 15 to 28 nucleotides in length and more preferably comprises at least 6 of the 7 nucleotides present in the seed sequence, i.e. the actual human derived sequence of the miRNAs as described herein.

Accordingly, a preferred miRNA-xx molecule or equivalent or mimic or isomiR thereof comprises at least 6 of the 7 nucleotides present in the seed sequence identified as any one of SEQ ID NOs: 1 to 4, and more preferably has a length of at least 6, 7, 8, 9, 10, 11, 12, 13, 14, 15, 16, 17, 18, 19, 20, 21, 22, 23, 24, 25, 26, 27, 28, 29, 30 nucleotides or more.

A source of a miRNA molecule or of an equivalent or a mimic or an isomiR thereof may be a single stranded optionally within a hairpin like structure, a double stranded RNA or a partially double stranded RNA or may comprise three strands, an example of which is described in WO2008/10558. As used herein partially double stranded refers to double stranded structures that also comprise single stranded structures at the 5'and/or at the 3' end. It may occur when each strand of a miRNA molecule does not have the same length. In general, such partial double stranded miRNA molecule may have less than 75% double stranded structure and more than 25% single stranded structure, or less than 50% double stranded structure and more than 50% single stranded structure, or more preferably less than 25%, 20 % or 15% double stranded structure and more than 75%, 80%, 85% single stranded structure.

Alternatively, a source of a miRNA molecule or of an equivalent or a mimic or an isomiR thereof is a DNA molecule encoding a precursor of a miRNA molecule or of an equivalent or a mimic or an isomiR thereof. The invention encompasses the use of a DNA molecule encoding a precursor of a miRNA molecule that has at least 70% identity with said sequence as identified in SEQ ID Nos 1 to 4. Preferably, the identity is at least 75%, 80%, 85%, 90%, 95%, 97%, 98%, 99% or 100%.

In the present invention the term "squamous cell carcinoma" or "SCC" shall include all different types of cancer that begin in squamous cells, such as head and neck cancer or a preneoplastic mucosal change, and particularly relate to cutaneous squamous cell carcinoma (cSCC) in a subject that suffers from a dysfunction or loss of the structural extracellular matrix protein collagen type VII, for example caused by mutations within the COL7A1 gene, wound healing disorders, or from a severe recessive subtype of dystrophic epidermolysis bullosa (RDEB).

In the present invention, the risk of a subject to suffer from squamous cell carcinoma is diagnosed. Preferably, the risk as diagnosed according to the present invention is based on the assessment of the relative risk as determined through detecting the expression level of at least one miRNA as described herein in a sample and comparing it to a control, such as the expression level in healthy donors. Furthermore, the invention further comprises detecting the expression level of miR-29c-5p as a stable reference miRNA and/or 5S-rRNA as a small reference RNA as a control. A change of the expression level indicates a change in the risk compared to the control, a decrease of the expression level of miR-301a-3p and/or miR-1260a when compared to healthy donors indicates an increased risk of said subject to suffer from squamous cell carcinoma, and wherein an increase of the expression level of miR-187-3p and/or miR-27b-3p when compared to healthy donors indicates an increased risk of said subject to suffer from squamous cell carcinoma.

Preferred is a method according to the present invention, wherein a decrease of the expression level of miR-301a-3p and/or miR-1260a when compared to healthy donors indicates an increased risk of said subject to suffer from squamous cell carcinoma, and wherein an increase of the expression level of miR-187-3p and/or miR-27b-3p when compared to healthy donors indicates an increased risk of said subject to suffer from squamous cell carcinoma.

As mentioned above, further preferred is a method according to the present invention, further comprising detecting the expression level of miR-29c-5p as a stable reference miRNA and/or 5S-rRNA as a small reference RNA as a control.

In addition to the above miRNAs, the invention also encompassed a method according to the present invention, further comprising detecting the expression level of at least one of the miRNA molecules, an isomiR, or a precursor thereof selected from the group consisting of miR-769-3p, miR-22-5p, miR125a-3p, miR-198-3p, miR-95-3p, miR-193b-3p, miR-342-3p, miR-125a-5p2, miR-125a-3p4, miR-941, miR-125a-5p5, miR-10a-5p, let-7e-5p6, miR-34a-5p7, miR-193b-5p8, miR-125a-3p9, miR-1307-5p, miR-34a-5p10, miR-10a-3p, and miR-125a-5p. These additional miRNAs will advantageously add further relevance to the panel or set of miRNAs as above, i.e., miR-187-3p, and at least one of miR-27b-3p, miR-301a-3p and/or miR-1260a.

An important aspect of the invention is the non-invasive nature of obtaining the sample. Preferred is the method according to the present invention, wherein the sample comprises a liquid biopsy and/or swap sample, such as a wound swab sample, from said subject. Despite the inventors' previous report on abnormalities in miRNA expression of RDEB-SCC, the inventors could not find a convergent pattern matching the inventors' swab-derived data, neither in tumor cells nor in tumor cell-derived microvesicles. The method of the invention nevertheless also encompasses a pre-conditioned sample, such as to obtain the exosome preparation of the sample before the assay.

Another important aspect of the invention relates to the fact that the subject to be diagnosed suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

Another important aspect of the invention relates to a method according to the present invention, further comprising the (actual) step of diagnosing the subject to suffer from SCC in particular cutaneous squamous cell carcinoma (cSCC). As a result, an information is obtained whether the subject has developed SCC or not.

Any suitable method can be used in the method according to the present invention, in order to detect the expression level of said miRNA(s). There are a number of well-established miRNA detection methods that can be exploited depending on the comprehensiveness of the study (individual miRNA versus multiplex analysis), the availability of the sample and the location and intracellular concentration of miRNAs (see, for example, Yaylak B, Akgül B. Experimental MicroRNA Detection Methods. Methods Mol Biol. 2022;2257:33-55. doi: 10.1007/978-1-0716-1170-8_2. PMID: 34432272). miRNA expression levels can be studied by several methods: microarray analysis, real-time PCR, Northern blots, miRNA sequencing, in situ hybridization, and solution hybridization, and the most prominent examples are miRNA sequencing, solution hybridization assays and quantitative polymerase chain reaction (qPCR). Another important aspect of the invention relates to a method according to the present invention, wherein the changed expression level of said miRNA, as compared to healthy donors, shows a significant (FDR < 0.05) differential abundance or expression level (min. 2-fold) in said sample vs healthy control. This can be determined as described herein, for example using a computer program or app.

Another important aspect of the invention relates to a method according to the present invention, wherein the sample from the subject is obtained from said subject either before, during or after a treatment of said subject against wound healing disorders, RDEB and/or SCC, in particular cSCC. This relates to a monitoring of the effect of a treatment of the SCC (such as a chemotherapy) and/or shows the effectiveness of a surgical removal of the SCC cells from the subject or lesion.

An important aspect of the invention then relates to a diagnostic composition comprising a sample obtained from a subject at risk to suffer from squamous cell carcinoma (SCC) comprising miR-187-3p molecule, an isomiR, or a precursor thereof. Preferably, the diagnostic composition according to the present invention further comprises at least one of miR-27b-3p, miR-301a-3p, or miR-1260a molecule, an isomiR, Preferably, the diagnostic composition according to the present invention further comprises miR-29c-5p as a stable reference miRNA and/or 5S-rRNA as a small reference RNA as a control.

The diagnostic composition according to the present invention can be suitably prepared by obtaining a sample form a subject, and optionally admixing the sample with suitable additional components for the diagnostic assay. The diagnostic composition of the invention may also comprise pre-conditioned components, such as an exosome preparation of the sample as taken from the subject.

As mentioned above, preferably, the diagnostic composition according to the present invention further comprises at least one of miRNA molecules, an isomiR, or a precursor thereof selected from the group consisting of miR-769-3p, miR-22-5p, miR125a-3p, miR-198-3p, miR-95-3p, miR-193b-3p, miR-342-3p, miR-125a-5p2, miR-125a-3p4, miR-941, miR-125a-5p5, miR-10a-5p, let-7e-5p6, miR-34a-5p7, miR-193b-5p8, miR-125a-3p9, miR-1307-5p, miR-34a-5p10, miR-10a-3p, and miR-125a-5p.

More preferably, in the diagnostic composition according to the present invention the sample comprises a liquid biopsy and/or swap sample, such as a wound swab sample, from said subject.

Preferably, the diagnostic composition according to the present invention is derived from a subject suffering from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

Another important aspect of the invention relates to a diagnostic composition according to the present invention, wherein the sample from the subject was obtained from said subject either before, during or after a treatment of said subject against wound healing disorders, RDEB and/or SCC, in particular cSCC. These samples relate to a monitoring of the effect of a treatment of the SCC (such as a chemotherapy) and/or shows the effectiveness of a surgical removal of the SCC cells from the subject or lesion.

Another important aspect of the invention relates to a kit for in vitro assessment of the risk that a subject suffers from squamous cell carcinoma (SCC), comprising buffers and reagents to perform a method according to the present invention as described above and herein, optionally together with instructions for performing said method.

Preferred is the kit according to the present invention wherein said kit comprises suitable buffers and reagents to perform quantitative polymerase chain reaction (qPCR), microarray analysis, real-time PCR, Northern blots, in situ hybridization, miRNA sequencing, and solution hybridization, and/or the kit further comprising reagents needed to obtain the exosome preparation of the sample.

Another important aspect of the invention relates to the use of the kit according to the present invention for diagnosing a subject to suffer from SCC, in particular cutaneous squamous cell carcinoma (cSCC), in particular according to a method according to the present invention as described herein.

Another important aspect of the invention relates to a computer program for identifying a subject's risk to suffer from SCC, in particular cutaneous squamous cell carcinoma (cSCC), in particular as determined according to a method according to the present invention, wherein said program is preferably implemented to run on a computer. Preferred is the computer program according to the present invention, which is a Shiny app.

Another important aspect of the invention relates to a method, such as a screening method, for identification of a substance capable of preventing, treating, reverting and/or delaying a condition or disease associated with a squamous cell carcinoma such as head and neck cancer or a preneoplastic mucosal change in a subject, the method comprising the steps of:
(a) providing a test cell population capable of expressing a miR-187-3p, miR-27b-3p, miR-301a-3p, and/or miR-1260a molecule or equivalent thereof, preferably the test population comprises cells, more preferably the test cell population comprises mammalian cells, even more preferably human cells;
(b) contacting or incubating the test cell population with the substance;
(c) determining the expression level of said miR-187-3p, miR-27b-3p, miR-301a-3p, and/or miR-1260a molecule or equivalent thereof or the activity or steady state level of said miR-187-3p, miR-27b-3p, miR-301a-3p, and/or miR-1260a molecule or equivalent thereof in the test cell population contacted or incubated with the substance;
(d) comparing the expression, activity or steady state level determined in (c) with the expression, activity or steady state level of said miR-187-3p, miR-27b-3p, miR-301a-3p, and/or miR-1260a molecule or equivalent in a test cell population that is not contacted with the substance; and,
(e) identifying a substance that produces a difference in expression level, activity or steady state level of said miR-187-3p, miR-27b-3p, miR-301a-3p, and/or miR-1260a molecule or equivalent, between the test cell population that is contacted with the substance and the test cell population that is not contacted with the substance.

Yet another important aspect of the invention then relates to a method of preventing or treating squamous cell carcinoma (SCC), in particular cSCC, in a subject, comprising the steps of performing method according to the present invention to determine if the subject has an increased probability of suffering or is suffering from squamous cell carcinoma (SCC), in particular cSCC, and selecting an appropriate prevention or therapy for the subject based on the determination, such as a chemotherapy and/or a surgical removal of the SCC cells from the subject or lesion, or identifying of a substance capable of preventing, treating, reverting and/or delaying a condition or disease associated with a squamous cell carcinoma according to the present invention, and selecting an appropriate prevention or therapy for the subject based on the substance as identified.

Preferred is the method according to the present invention, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

Preferred is the method according to the present invention, wherein the sample from the subject was obtained from said subject either before, during or after a treatment of said subject against wound healing disorders, RDEB and/or SCC, in particular cSCC, again relating to a monitoring of the effect of a treatment of the SCC.

In another aspect thereof, an embodiment of the method for diagnosing a disease or condition associated with a squamous cell carcinoma such as head and neck cancer or a preneoplastic mucosal change in a subject, the method according to the present invention comprises the steps of: (a) determining the expression level of miR-187-3p molecule, an isomiR, or a precursor thereof in a sample obtained from a subject at risk to suffer from squamous cell carcinoma, and optionally further determining the expression level of at least one of miR-27b-3p, miR-301a-3p, or miR-1260a molecule, an isomiR, or a precursor thereof; (b) comparing the expression level of said molecule as defined in (a) with a reference value for the expression level of said molecule, the reference value preferably being the average value for the expression level of said molecule in a healthy subject, and (C) optionally, concluding on squamous cell carcinoma such as head and neck cancer or a preneoplastic mucosal change in the subject based on said comparing. Preferably, the method according to the present invention further comprises the detection of miR-29c-5p as a stable reference miRNA and/or 5S-rRNA as a small reference RNA as a control.

Preferred is a method according to the present invention, wherein the expression level of said miRNA is determined indirectly by quantifying the amount of the nucleotide sequence, for example as described above. Further preferred, the expression level is determined ex vivo in a sample obtained from the subject.

In order to improve cancer diagnosis, the identification of tumor-specific biomarkers bears great potential for supporting (early) detection and/or treatment monitoring in RDEB-patients. Micro-RNAs (miRNAs) have been shown to hold this potential in the context of several malignancies, and their dysregulation has been associated with tumor development and progression at various levels, affecting tumor hallmarks like the induction of epithelial-to-mesenchymal transition (EMT), or conferring stemness-associated characteristics (Calin et al. 2019, Calin et al. 2006, Rupaimoole et al. 2016, Wimmer et al. 2020, Hanahan et al. 2011). In general, miRNAs regulate protein expression at the post-transcriptional level and a classification of distinct miRNAs as onco-, metasta-, and tumor suppressor-miRs reflects their association with malignant processes (Bartel et al. 2004, Calin et al. 2006). Tumor-specific expression of miRNAs and their active or passive release into the extracellular matrix (ECM) and circulation upon cell death or via microvesicles underlines their attractiveness to be exploited as biomarkers, as they can be extracted from well accessible sources, and particularly from cutaneous wounds. Further, their stability and relative abundance renders them an interesting alternative to e.g. cell-free DNA or circulating tumor cells (Schwarzenbach et al. 2014).

In the context of the present invention, the inventors aimed to identify a miRNA signature indicative of malignancy in patients with RDEB. To this end the inventors extracted miRNAs from cutaneous wounds, which were designated either as tumor lesions, based on a positive histopathology report, or otherwise as "controls". Remarkably, the samples were collected from skin lesions using a non-invasive swabbing method established for such an application for the first time. State-of-the-art prediction models were trained using a miRNA-seq dataset generated from isolated miRNA and the resulting signature and models were tested on a large lesion from a patient who underwent extensive surgery to remove suspicious lesions with indistinct tumor boundaries. A wound map was created that included more than twenty sites with images for visual inspection, swab specimen for miRNA-based tumor prediction, and tissue biopsies to confirm clinical diagnosis. A head-to-head comparison between visual inspection performed by trained dermatologists and miRNA-based models suggests that the inventors' proposed novel non-invasive method has the potential to serve as useful diagnostic support in monitoring large chronic wound areas that are prone to developing tumors for early tumor detection in patients at high risk. In addition, the feasibility of transferring the swab/miRNA-based tumor prediction model from miRNA-seq to a simple and fast qPCR assay was assessed and a concept for a clinical path forward is presented.

In patients with RDEB, SCCs arise frequently and at particularly young age (Tartaglia et al., 2021, PMID: 34065916, Tang et al., PMID: 33849616). According to the guideline on the management of SCCs in patients with EB, surveillance is recommended to start at an age of 10 years and screening should take place every six to twelve months, after the first SCC has arisen even every three month. (Mellerio et al. 2015). Patients usually develop several primary tumors that frequently resemble nonmalignant lesions, thus, visual inspection requires a lot of experience in order to identify clinically suspicious lesions for further histopathologic examination by punch biopsy. The attending physicians have to balance the risk of taking too few biopsies and thus increasing the risk of missing a tumor with the drawback that biopsies represent an additional burden for the patients, as wounds often do not heal satisfactorily. At the same time, biopsies cannot cover a suspicious area to the full extent. Therefore, a non-invasive tool such as swabbing would facilitate pinpoint identification of malignant tissue, as multiple samples can be collected without further harming patients. Profiling these samples will support physicians in deciding where additional biopsies should be taken, particularly if regions can be identified that were not clearly suspicious. (Mellerio et al. 2015, Condorelli et al. 2019, PMID: 31739489). Thus, the inventors' method offers the possibility of detecting malignant transformations in large wounds and skin areas and reducing the burden to patients.

Exploitation of miRNAs as biomarkers have gained attention as they are abundant in a variety of body fluids and have high stability, owing to their incorporation into microvesicles or complexation with RNA-binding proteins, which protects them from nuclease degradation (Schwarzenbach et al. 2014, Cortez et al. 2011). Both, active and passive release by apoptotic or necrotic events from tumor cells, contribute to changes in circulating miRNA levels that may indicate an altered physiological status. (Schwarzenbach et al. 2014, Mitchell et al. 2008).

The clinical utility of miRNAs as biomarkers has recently been demonstrated in landmark studies. In Japan, screening of more than 10,000 serum samples from patients with malignant diseases resulted in a diagnostic test for early detection of 13 different types of cancer (Matsuzaki et al. 2020). An effort in developing a miRNA panel for lung cancer screening based on a retrospective analysis of the randomized Multicenter Italian Lung Detection (MILD) trial has meanwhile been further advanced to a large prospective trial (BioMILD) with important implications on personalizing screening intervals (Sozzi et al. 2014, Pastorino et al. 2022). Alterations in miRNA profiles are frequently associated with various disease states, including tumorigenesis, in which miRNAs play an important biological and clinical role by regulating gene expression and thereby tuning many important cellular pathways (Bartel et al. 2004, Calin et al. 2002, Croce et al. 2009, Peng et al. 2016). Recently, an aberrant miRNome was also confirmed in RDEB tumors (Wimmer et al. 2020). Despite the inventors' previous report on abnormalities in miRNA expression of RDEB-SCC, the inventors could not find a convergent pattern matching the inventors' swab-derived data, neither in tumor cells nor in tumor cell-derived microvesicles.

For this reason, the inventors argue that the four proposed signature miRNAs are unlikely to originate directly from tumor cells, but rather reflect tumor-driven transformations of the tumor microenvironment (TME). This is in line with reports linking the dynamic interaction between the tumor and it's TME to compositional changes in circulating miRNAs, significantly influenced by non-tumor cells (Cui et al. 2019, Boeri et al. 2012, Mensah et al. 2017, Fortunato et al. 2019). In fact, a recent study investigating the origin of a panel of 24 miRNAs that has been shown to be effective in predicting lung cancer in clinical trials found that granulocytes and platelets were major contributors (Pastorino et al. 2022, Fortunato et al. 2019). A closer examination of the inventors' miRNA panel suggests that only miR-27b-3p is expressed at higher levels in tumor cells (Supp. Fig. 7A-B). A dual role as either an onco-miRNA or tumor suppressor has been documented for miR-27b-3p in various cancers, affecting many tumor-associated pathways, such as the VEGF/VEGFR2 - MAPK signaling axis, which shows alterations in tumor tissue of head and neck, organ transplant recipients and RDEB-SCC patients (Ding et al. 2017, Zauner et al. 2022/01). Although there are currently no specific reports on miR-1260a in cutaneous SCC, it has been shown that T-cell activation leads to extensive change in miRNA expression including miR-1260a and T-helper-derived exosomes consistently contain miR-1260a (Rodríguez-Galán et al. 2021, Torri et al. 2017). In addition, a difference in the miRNA-cargo of vesicles derived from neutrophils, which are either residing at or in the progress of migrating to inflammatory foci was observed, highlighting changes in miR-1260a levels (Youn et al. 2021). It is further documented that miR-187 is involved in controlling cytokine expression in phagocytes in an II,-10 dependent manner, and that miR-301a is triggered in fibroblasts by multiple inflammatory stimuli (Rossato et al. 2012, Wang et al. 2020). Taken together, this supports the reasonable conjecture that tumor-associated changes in cellular composition and adaptations in molecular programs contribute to the observed swab-based miRNA signature.

This invention provides the development of a minimally-invasive diagnostic test to complement current standards of care in monitoring chronics wounds in the particularly challenging setting of ulcerating and scarring skin of RDEB patients and ultimately aims to support early detection of life-threatening tumors.

Here, the inventors investigated the potential of miRNAs to be used as diagnostic markers for spatially resolved identification of SCCs by systematically profiling > 50 skin lesions sampled from 12 RDEB-patients. The inventors' results illustrate that a small set of only four miRNAs derived from training tree- and rule-based classification models was sufficient to distinguish SCC from non-cancerous wounds with > 90% accuracy and that tumor lesions could be detected before macroscopic visibility. In addition, the inventors demonstrated for the first time that more than 260 miRNAs can be robustly extracted from cutaneous wound swabs and that a prediction model trained on miRNA-seq data can be transferred to qPCR assay by identifying stable reference miRNAs.

The inventors report a hitherto unexplored technique of swab-based miRNA extraction from RDEB lesions with the potential to delineate tumors from a background of ulcerating and fibrotic scarring EB skin. The inventors propose a combination of four signature miRNAs able to predict tumor samples, outperforming a single miRNA predictor, chosen based on recent data showing deregulation of miR-10 family members in RDEB tumors (Wimmer et al. 2020).

In summary, the inventors propose a simple, non-invasive sampling technique to detect RDEB-SCCs at an early stage that facilitates the concomitant analysis of numerous suspect skin areas, without inflicting significant additional trauma and pain to patients.

The present invention relates to the following items:
Item 1: A method for diagnosing the risk of a subject to suffer from squamous cell carcinoma (SCC), comprising detecting the expression level of the miR-187-3p molecule, an isomiR, or a precursor thereof in a sample obtained from said subject, wherein a changed expression level of said miRNA, as compared to healthy donors, indicates an increased risk of said subject to suffer from squamous cell carcinoma.
Item 2. A method according to Item 1, further comprising detecting the expression level of at least one of the miR-27b-3p, miR-301a-3p, or miR-1260a, an isomiR, or a precursor thereof in said sample obtained from said subject, wherein a changed expression level of said at least one miRNA, as compared to healthy donors, further indicates an increased risk of said subject to suffer from squamous cell carcinoma.
Item 3. A method according to Item 1 or 2, wherein a decrease of the expression level of miR-301a-3p and/or miR-1260a when compared to healthy donors indicates an increased risk of said subject to suffer from squamous cell carcinoma, and wherein an increase of the expression level of miR-187-3p and/or miR-27b-3p when compared to healthy donors indicates an increased risk of said subject to suffer from squamous cell carcinoma.
Item 4. The method according to any one of Items 1 to 3, further comprising detecting the expression level of miR-29c-5p as a stable reference miRNA and/or 5S-rRNA as a small reference RNA as a control.
Item 5. The method according to any one of Items 1 to 4, further comprising detecting the expression level of at least one of the miRNA molecules, an isomiR, or a precursor thereof selected from the group consisting of miR-769-3p, miR-22-5p, miR125a-3p, miR-198-3p, miR-95-3p, miR-193b-3p, miR-342-3p, miR-125a-5p2, miR-125a-3p4, miR-941, miR-125a-5p5, miR-10a-5p, let-7e-5p6, miR-34a-5p7, miR-193b-5p8, miR-125a-3p9, miR-1307-5p, miR-34a-5p10, miR-10a-3p, and miR-125a-5p.
Item 6. The method according to any one of Items 1 to 5, wherein the sample comprises a liquid biopsy and/or swap sample, such as a wound swab sample, from said subject.
Item 7. The method according to any one of Items 1 to 6, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).
Item 8. The method according to any one of Items 1 to 7, further comprising the step of diagnosing the subject to suffer from SCC, in particular cutaneous squamous cell carcinoma (cSCC).
Item 9. The method according to any one of Items 1 to 8, wherein detecting the expression level of said miRNA comprises quantitative polymerase chain reaction (qPCR).
Item 10. The method according to any one of Items 1 to 9, wherein the changed expression level of said miRNA, as compared to healthy donors, shows a significant (FDR < 0.05) differential abundance (min. 2-fold) in said sample vs healthy control.
Item 11. The method according to any one of Items 1 to 10, wherein the sample from the subject is obtained from said subject either before, during or after a treatment of said subject against wound healing disorders, RDEB and/or SCC, in particular cSCC.
Item 12. A diagnostic composition comprising a sample obtained from a subject at risk to suffer from squamous cell carcinoma (SCC) comprising miR-187-3p molecule, an isomiR, or a precursor thereof.
Item 13. The diagnostic composition according to Item 12, further comprising at least one of miR-27b-3p, miR-301a-3p, or miR-1260a molecule, an isomiR, or a precursor thereof.
Item 14. The diagnostic composition according to Item 12 or 13, further comprising miR-29c-5p as a stable reference miRNA and/or 5S-rRNA as a small reference RNA as a control.
Item 15. The diagnostic composition according to any one of Items 12 to 14, further comprising at least one of miRNA molecules, an isomiR, or a precursor thereof selected from the group consisting of miR-769-3p, miR-22-5p, miR125a-3p, miR-198-3p, miR-95-3p, miR-193b-3p, miR-342-3p, miR-125a-5p2, miR-125a-3p4, miR-941, miR-125a-5p5, miR-10a-5p, let-7e-5p6, miR-34a-5p7, miR-193b-5p8, miR-125a-3p9, miR-1307-5p, miR-34a-5p10, miR-10a-3p, and miR-125a-5p.
Item 16. The diagnostic composition according to any one of Items 12 to 15, wherein the sample comprises a liquid biopsy and/or swap sample, such as a wound swab sample, from said subject.
Item 17. The diagnostic composition according to any one of Items 12 to 16, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).
Item 18. The diagnostic composition according to any one of Items 12 to 17, wherein the sample from the subject was obtained from said subject either before, during or after a treatment of said subject against wound healing disorders, RDEB and/or SCC, in particular cSCC.
Item 19. A kit for in vitro assessment of the risk that a subject suffers from squamous cell carcinoma (SCC), comprising buffers and reagents to perform a method according to any one of claims 1 to 11, optionally together with instructions for performing said method.
Item 20. The kit according to Item 19, wherein said kit comprises suitable buffers and reagents to perform quantitative polymerase chain reaction (qPCR), and/or the kit further comprising reagents needed to obtain the exosome preparation of the sample.
Item 21. Use of the kit according to Item 19 or 20 for diagnosing a subject to suffer from SCC, in particular cutaneous squamous cell carcinoma (cSCC), in particular according to a method according to any one of Items 1 to 11.
Item 22. A computer program for identifying a subject's risk to suffer from SCC, in particular cutaneous squamous cell carcinoma (cSCC), in particular as determined according to a method according to any one of Items 1 to 11, wherein said program is preferably implemented to run on a computer.
Item 23. The computer program according to Item 22, which is a Shiny app.
Item 24. A method of preventing or treating squamous cell carcinoma (SCC), in particular cSCC, in a subject, comprising the steps of performing method according to any one of Items 1 to 11, to determine if the subject has an increased probability of suffering or is suffering from squamous cell carcinoma (SCC), in particular cSCC, and selecting an appropriate prevention or therapy for the subject based on the determination.
Item 25. The method according to Item 24, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).
Item 26. The method according to Items 25 or 26, wherein the sample from the subject was obtained from said subject either before, during or after a treatment of said subject against wound healing disorders, RDEB and/or SCC, in particular cSCC.

The invention will now be described further in the following examples with reference to the accompanying figures, nevertheless, without being limited thereto. For the purposes of the present invention, all references as cited are incorporated by reference in their entireties.

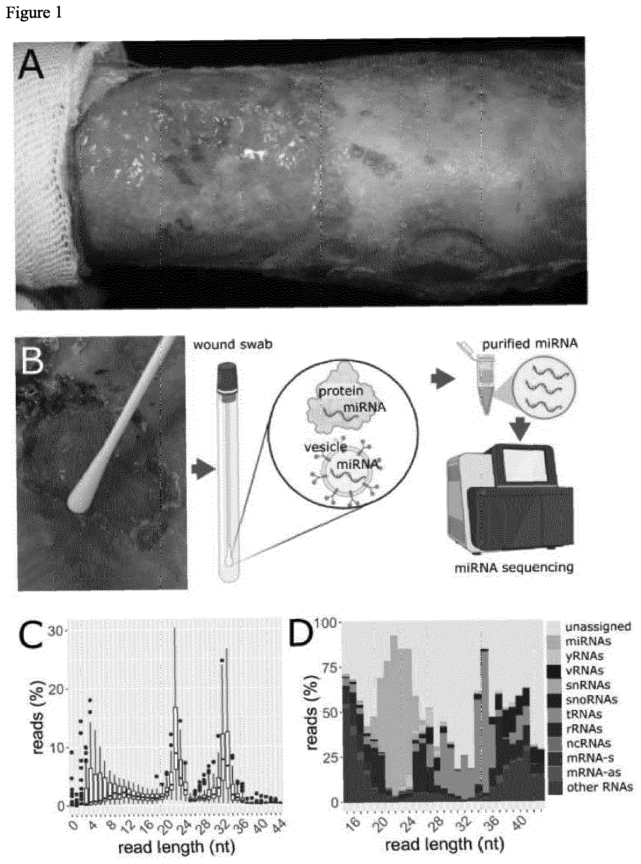

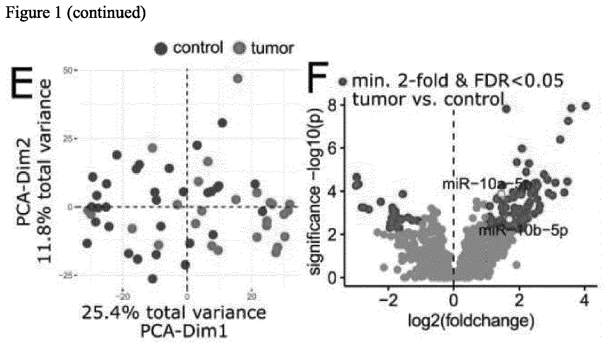

Figure 1 shows that wound swabs contain miRNAs with specific patterns in tumor lesions. (A) Typical chronic RDEB wound (patient #16) with several tumor-suspicious areas. (B) Schematic shows generation of swab-derived miRNA dataset. miRNA sequencing libraries were prepared from miRNAs isolated from swab specimens of RDEB wounds as well as confirmed tumor lesions, and subsequently sequenced on an Illumina platform (scheme created with BioRender.com). (C) Read length distribution of all sequenced samples, clearly indicating a peak at the expected length for miRNAs ranging between 20-24 nucleotides (nt). (D) Composition of annotated reads at specific read lengths with predominant assignment of reads at 20-24 nt to miRNAs (representative sample: 168614). (E) PCA conducted on centered, TMM normalized, log2 + 1 transformed miRNAs shows a tendency of tumor samples clustering to the right side with the first two dimensions capturing ~37% of total variance in miRNA abundance. (F) Volcano plot exemplifies a set (red dots) of miRNAs with significant (FDR < 0.05) differential abundance (min. 2-fold) in tumor vs control samples.

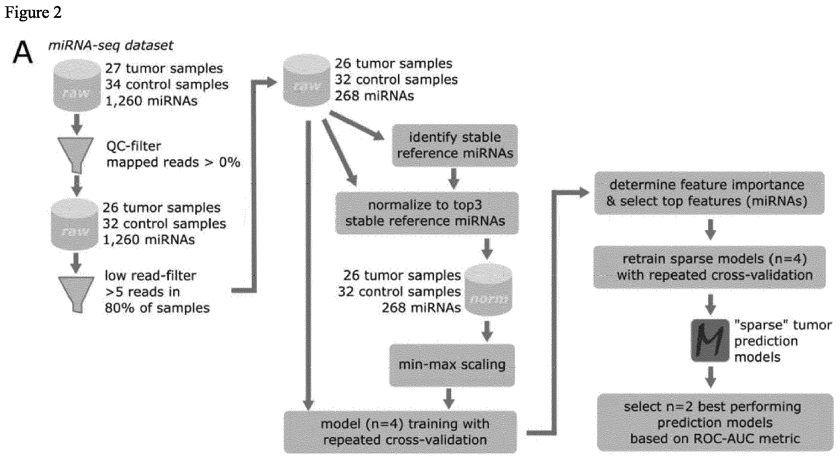

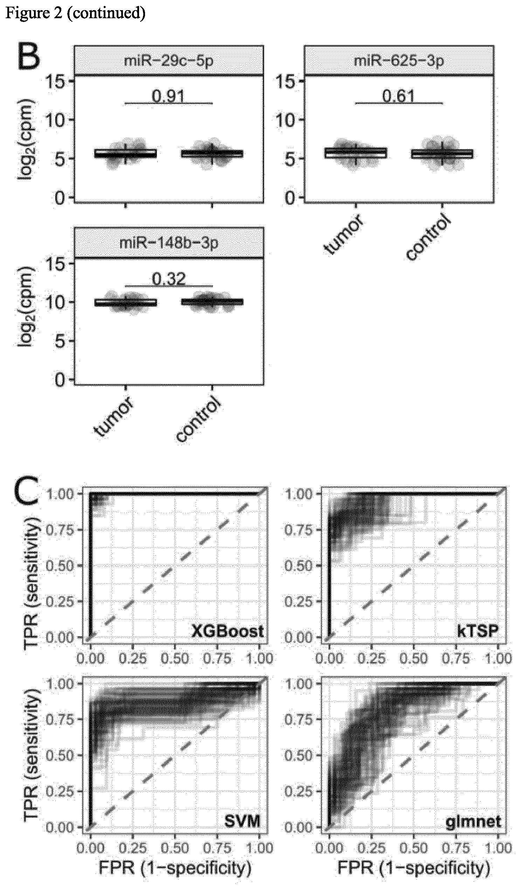

Figure 2 shows the training and performance evaluation of miRNA-based tumor prediction models. (A) Workflow outlines data analysis strategy starting with pre-processing of swab miRNA-seq dataset including relevant filter steps, stable reference miRNA selection for data normalization, followed by training of four complementary prediction models, feature importance analysis and retraining of "sparse" model, and finally the selection of two top performing models. (B) Boxplots illustrate the inventors' choice of "stable reference miRNAs", each dot representing one swab sample, associated to either the control or tumor group (x-axis) and log2-transformed, TMM normalized miRNA abundance in counts per million (cpm, y-axis), significance test: unpaired, two-sample nonparametric Wilcox. (C) Graphs show overlay of ROC curves demonstrating the prediction performance of four "sparse" models upon 100 times re-sampling from the complete pool of control (n = 32) and tumor (n = 24) samples, which were used to initially train the models. TPR: true positive rate, FPR: false positive rate, dashed line indicates the performance of random chance prediction.

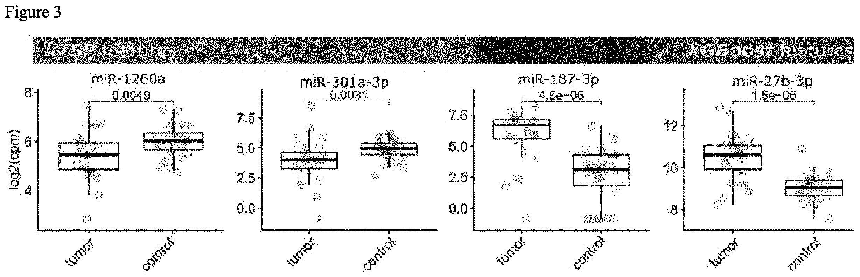

Figure 3 shows the signature miRNAs for swab-based tumor prediction according to the invention. Boxplots show miRNAs used in either kTSP prediction model (red label: miR-1260a, miR-301a-3p and miR-187-3p) or XBGoost (blue label: miR-187-3p and miR-27b-3p). The predictions models have one shared miRNA (dark blue label: miR-187-3p). Each dot represents one sample from the control or tumor group (x-axis). Abundance of respective miRNAs is presented as log2-transformed, TMM normalized counts per million (cpm, y-axis). Significance test: unpaired, two-sample nonparametric Wilcox.

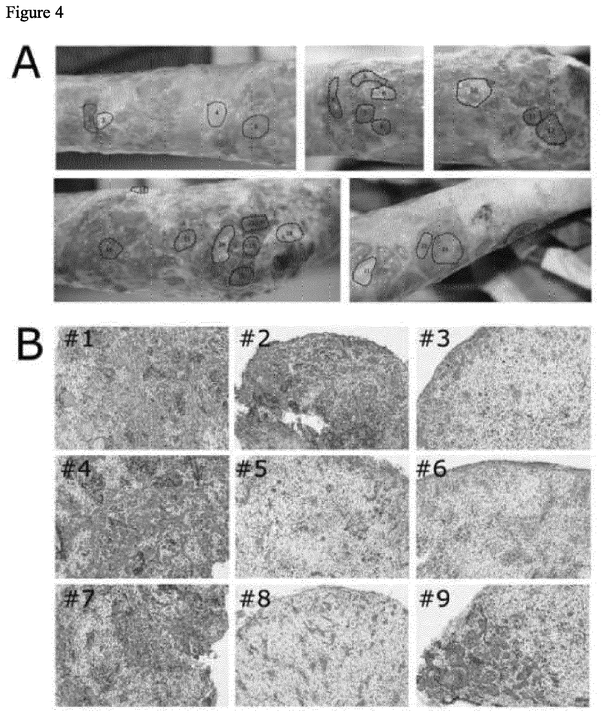

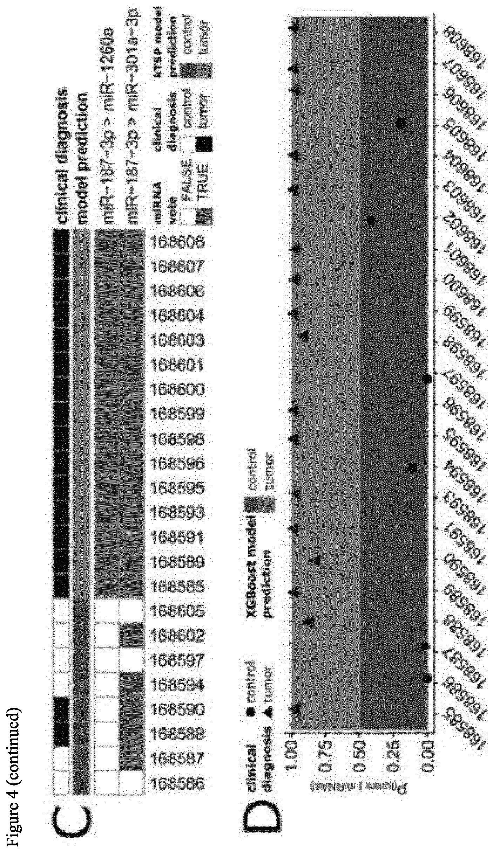

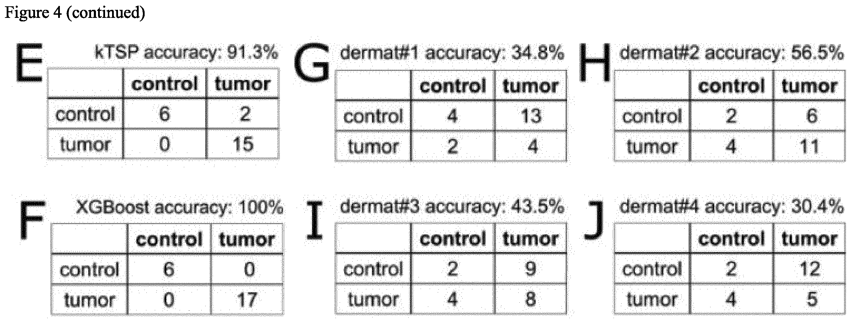

Figure 4 shows the prediction performance in wound mapping experiment. (A) Image shows sites (marked by dashed lines) where swabs and tissue biopsies were taken (patient #16). In total in 23 samples (wound mapping locations #1-#24) were collected from several chronic wound areas from one patient (patient #16). (B) H&E staining of skin biopsies (wound mapping locations #1-#9), examined by a trained dermato-pathologist to confirm clinical status (tumor vs non-tumor lesion). (C) Voting scheme of kTPS tumor prediction model. If both comparisons (miRNA votes - gray box: true, white box: false) were true, the algorithm predicted the sample as tumor (kTSP model prediction - red box: tumor vs blue box: control). The clinical diagnosis is shown as reference (white box: control, black box: tumor). (D) The sparse XGBoost prediction model provides a probability p of a sample being a tumor given the miRNA which are as features. If p(tumor|miRNAs) > 0.5 the sample was called a tumor (light gray), otherwise control (dark gray). The shape of the sample reflects the clinical diagnosis (tumor: triangle, control: dot). (E-F) Confusion matrix presents sparse model predictions for each sample in rows and clinical diagnosis in columns. Threshold for calling a sample tumor required both votes for (E) kTSP and >50% tumor probability in (F) XGBoost. (G-J) Four trained dermatologists visually inspected each spot on the wound map based on the images (A) and labeled the spots as tumor or control. (E-J) Prediction accuracy was calculated as a ratio of the sum of true positives and true negatives vs the sum of true positives, true negatives, false positives and false negatives. (E-J) Columns: clinical diagnosis; rows: model prediction.

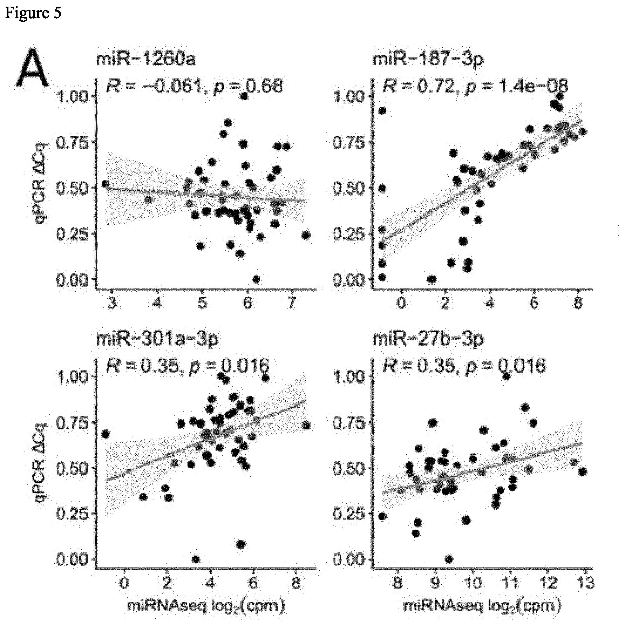

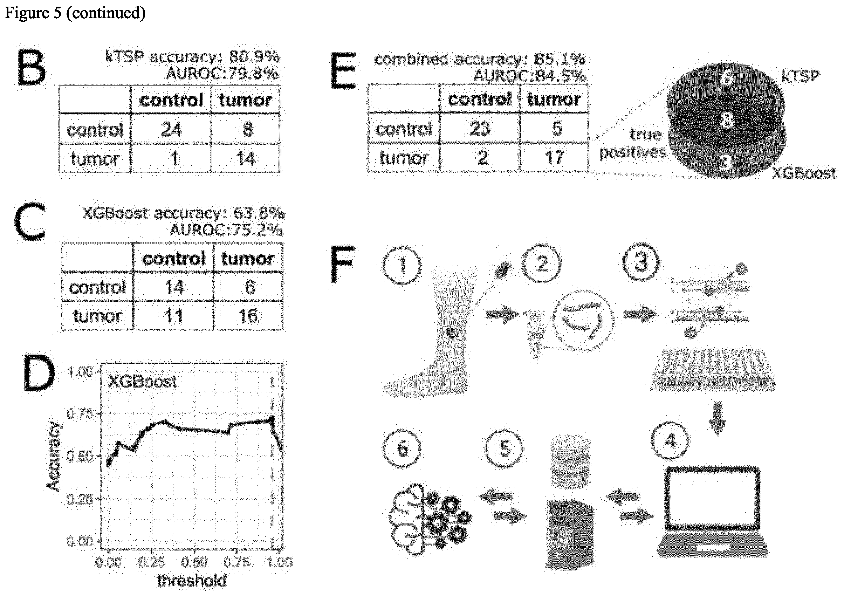

Figure 5 shows the qPCR-based tumor prediction according to the present invention. (A) Scatterplots compare the relative abundance of four signature miRNAs which were detected either by a qPCR assay (y-axis) or miRNA-seq (x-axis). Regression lines indicate strength of correlation (R, statistical test: Pearson) with gray ribbon bands showing the 95% confidence interval. ΔCq: relative to the mean of miR-29c-5p (stable reference miRNA) and 5S-rRNA (small reference RNA), miRNA-seq data represents log2-transformed, TMM normalized counts per million (cpm). (B-C) Confusion matrices contain model predictions of in total 47 samples analyzed by qPCR. Threshold for calling a sample tumor was set to min. one tumor vote out of two for (B) kTSP and >50% tumor probability in (C) XGBoost. (B-E) Columns: clinical diagnosis; rows: model prediction. Prediction accuracy was calculated as a ratio of the sum of true positives and true negatives vs the sum of true positives, true negatives, false positives and false negatives. (D) Plot indicates a sweet spot around 96% threshold (dashed line) to gain max. accuracy for XBGoost predictions. (E) The predictions of kTSP (threshold min. one tumor vote out of two) and sparse XGBoost (>96% tumor probability) were combined to call a sample a tumor if either of the two models labeled the sample as tumor. Venn diagram illustrates each individual model's contribution to true positive votes. (F) Drawing presents a preferred approach how a swab-based diagnostic assay is realized based on qPCR in combination with a browser app to analyze the data, schematic created with BioRender.com.

### Examples

### Material and methods

Samples from different types of lesions were included in the study, all derived from patients with RDEB who gave written informed consent. Ethical approval was granted by the ethics committee of the county of Salzburg (vote numbers: 415-E/1990/8-216, and 415-EP/73/192-2013). All procedures described in this study are in full accordance with Austrian legislation and with the Declaration of Helsinki. Lesions were classified as wounds (control) or tumor lesions if diagnosed as squamous cell carcinomas by histology.

### Swab specimen collection and miRNA isolation

Wounds were swabbed with viscose swabs for RNA collection (#300252, deltalab, Barcelona, Spain). The swab was gently rolled about 10 times over the lesion of interest and was then transferred into a 1.5 mL tube containing 700 µL Qiazol (#79306, Qiagen, Hilden, Germany), vortexed for 30 sec, incubated overnight at 4°C, and stored at -80°C until further processing. RNA was isolated using the miRNeasy mini kit (#217004, Qiagen, Hilden, Germany), following manufacturer's instructions. Small RNA concentrations were determined using a Qubit assay (Q32880, Thermo Fisher, Waltham, US) and samples stored at -80°C.

### miRNA sequencing

MiRNA sequencing library preparation was conducted using the QIAseq miRNA library kit (#331502, Qiagen, Hilden, Germany) for Illumina next generation sequencing and QIAseq miRNA NGS 48 Index kit (#331595, Qiagen, Hilden, Germany) following the manufacturer's instructions. Briefly, 5µL RNA were used as starting material. Library quality was assessed by Fragment Analyzer 5200 (Agilent, Santa Clara, US) and RT-PCR (Library Quant Kit KK4827, Roche, Basel, Switzerland). Sequencing was performed on Illumina NextSeq550 or NextSeq2000 instruments (> 75 cycles, single end reads, Illumina, San Diego, US) at the Vienna Biocenter (VBC) core facility.

### Pre-processing of miRNA-seq data and stable reference miRNA determination.

FastQ files were processed in sRNAtoolbox with default parameter settings (Aparicio-Puerta et al. 2019). In short, after adapter trimming, reads were aligned to the reference genome (GRCh38) and miRBase (v22) using bowtie seed alignment with seed length of 20 nts, minimum read length of 15 nts and restricting allowed mismatches to 1 nt. Subsequently reads counts for mature miRNAs from different genomic loci were aggregated and adjusted for multiple mappings. Samples with 0% reads mapped to mature miRNAs were labeled as low quality samples and removed from any further analysis. MiRNAs were normalized to compensate for differences in sequencing depth and library composition by either EdgeR's TMM to provide log2 transformed counts per million (EdgeR's cpm function) for the selection of stable reference miRNAs, PCA and signature miRNA boxplots, or by applying DESeq2 library-size normalization (Robinson et al. 2010, Love et al. 2014). PCA was conducted on centered, unscaled EdgeR normalized count data using R packages FactoMineR and factoextra (Lê et al. 2008, Love et al. 2014). Low count miRNAs were removed keeping miRNAs with more than 5 reads in at least 80% of samples per condition which were labeled as stably "detected" miRNAs. SRMs were defined by calculating the significance in differential miRNA abundance between tumor and control samples applying three complementary widely used methods offered by EdgeR's glmQLFit, limma's voom and DESeq2 (Robinson et al. 2010, Law et al. 2014, Love et al. 2014). The top three miRNAs of an intersection of miRNAs sorted by decreasing significance values were finally chosen as SRMs. Raw count data was normalized to SRMs by log2 transformation of all miRNAs and subsequent subtraction of the mean of the three SRMs from individual miRNAs.

### Model training and prediction performance evaluation

Model training was conducted on all samples and miRNAs which passed quality control and low count filter respectively within R's caret machine learning framework which provides specialized functions for model parameter tuning with repeated (n=30) 10-fold cross-validation (Kuhn et al. 2008). With the exception of the kTSP model, SRM normalized miRNA count data was min-max scaled to accommodate differences in scale between miRNA-seq and qPCR data and promote a transfer of prediction models between the different assays.

Parameter tuning was performed using caret's model implementation of XGBoost (xgbTree), glmnet (glmnet), SVM (svmLinear) all configured with AUROC as metric for finding optimal model parameters. Tune-lengths defining the grid search space were set depending on model complexity to either 4 for XGBoost and 5 for glmnet and SVM. Re-training of XGBoost, glmnet and SVM was executed on a restricted set of miRNAs to obtain "sparse" prediction models, again with repeated (n=30) 10-fold cross-validation. The restricted set of miRNAs was acquired by choosing the top 2 miRNAs from a list of most important miRNAs provided by caret's varImp function for XGBoost based on a sharp decline in a variable importance plot or the top 3 in case of continuous decline in variable importance for glmnet and SVM. As kTSP represents a parameter-free model with integrated feature selection as well as an inherent within-sample normalization procedure by considering informative miRNAs in relation to another one, model training was carried out with raw count data resulting in a parsimonious model ab initio. Model selection was based on the AUROC metric averaged over all 30 x 10-fold test-sets left out during model training for performance evaluation. ROC curves were generated for bootstrapped samples drawing from all samples with replacement by using caret's createResample algorithm and plotted as overlay to illustrate differences in mode performance.

### Visual inspection of case study lesions

For the case study, several pictures of the indexed lesions from the left arm were taken before tumor surgery and clinically suspicious regions characterized by their bumpy, flat, fibrotic etc. appearance were identified and annotated as either suspect tumors or chronic lesions (control) by a trained dermatologist. Areas were marked in a wound map provided to the surgeon for obtaining the samples including swabs and curettage during the tumor removal procedure.

### Histological staining

Tissue biopsies were fixed in buffered 4% paraformaldehyde and further processed for formalin fixed and paraffin embedding for subsequent hematoxylin and eosin staining as described in detail elsewhere (Wimmer et al. 2020). Imaging was conducted on a light microscope (Leica Microsystems, Wetzlar, Germany, City, Country) with a digital camera (Nikon, Minato, Japan) and processed with Nikon Imaging Software.

### qPCR assay

An locked-nucleic-acid (LNA)-based semi-quantitative real-time RT-PCR assay was performed on RNA isolated from wound swabs which was also used for miRNA-sequencing. Therefore, 2 µL of RNA was reverse transcribed into cDNA with a miRCURY LNA RT Kit (#339340, Qiagen, Hilden, Germany). Thereof 4 µL of 1:80 diluted cDNA were used in 10 µL reactions of miRCURY LNA SYBR^{®} Green PCR Kit (#3339347, Qiagen, Hilden, Germany) for real-time PCR amplification with LNA-enhanced primers (hsa-miR-1260a, hsa-miR-301a-3p, hsa-miR-187-3p, hsa-miR-27b-3p, 5S-rRNA and hsa-miR-29c-5p) pre-dispensed in readyto-use custom PCR panel 96-well plates on a C1000 / CFX-96 Bio-Rad Laboratories instrument (Hercules, US) following manufacturer's instructions. ΔCq were calculated following the procedure proposed by Livak and Schmittgen by subtracting the mean of both reference miRNAs, 5S-rRNA and hsa-miR-29c-5p (Livak et al. 2001). Normalized qPCR data was inverted by multiplication with negative one to enforce higher values to represent higher abundance and min-max scaled before they were further processed in correlation analysis and used as input for tumor prediction models.

### Data processing and Shiny app

All data was processed with the statistical software R (version 4.1.0) and corresponding packages as stated in the respective method sections (R et al. 2020). A Shiny app was developed in RStudio (version 1.4.1717) to demonstrate how qPCR data can be analyzed without the need of any special bioinformatic resources (Rstudio Team et al. 2021)

### Swabs are a suitable non-invasive method to sample cutaneous miRNAs with informative tumor-associated profiles.

In order to provide a non-invasive and well-tolerable method to sample material from RDEBpatient's skin and lesions, the inventors investigated whether standard viscose swabs can be used for sample collection, and subsequently to isolate and examine miRNAs. While swabs have been previously used to analyze proteins in diabetic ulcers (Schmohl et al. 2012), to collect bacteria from various sites (Guttmann-Gruber et al. 2018) and are currently used for the detection of viral RNA to diagnose SARS-CoV-2 infection (Bruce et al. 2020), to the inventors' knowledge they have not yet been utilized to analyze skin-derived miRNAs. Considering the presence of exosomes, proteins and cells in wound exudates and blister fluids (Lettner et al. 2015, Fang et al. 2018) it appears reasonable to assume that miRNAs will be detectable amongst wound-related molecules, since miRNAs are associated with the three major components: complexed with proteins like argonauts, or present within cells or exosomes (Zauner et al. 2022).

In the present experiment 61 lesions from 12 different RDEB patients were swabbed. In addition, tissue biopsies of lesions suspicious for the presence of SCCs were collected to obtain site-matched histopathology reports (Tab. 1, Fig. 1A, B). Total RNA was isolated from the wound swabs yielding respective concentrations between 0.080 and 12.10 ng·µL-1.

**Table 1: Sample composition**

| In total 61 swab specimens were obtained from 12 different patients of both sexes with a similar average age at sampling. | | |
|---|---|---|
| | control | tumor |
| # of patients | 8 | 4 |
| # of samples | 34 | 27 |
| female | 23 | 25 |
| male | 11 | 2 |
| <age> | 27.8 | 34.8 |

Illumina next generation sequencing of miRNA libraries showed a length distribution of sequence reads that indicated the presence of a 20-24 bp fraction, which is in accordance with the expected size distribution of miRNAs (Fig. 1C). Further examination of the read annotation composition demonstrated that the majority of 20-24 bp reads could be mapped to canonical miRBase (v22) reference sequences (Fig. 1D).

In addition, sequencing results showed a highly diverse set of miRNAs with on average 552 ± 171 (mean ± sd) different miRNAs detected per swab sample.

Thus, the ability to retrieve miRNAs from swab samples appeared to be very robust considering that 95% of all samples contained a fraction of reads (1.9 - 25.4%) which matched to reference sequences of mature miRNAs.

To track down possible miRNAs patterns in swab specimens from benign wounds and confirmed tumor lesions, the inventors performed principal component analysis (PCA) on centered and normalized log2-transformed count data. Indeed, a tendency towards a separation of tumor from control samples could be observed in several PCA dimensions (Fig. 1E), which supported the inventors' assumption that certain combinations of wound swab-derived miRNAs might be informative for recognizing the presence of tumors. Furthermore, differences in miRNA abundance between tumor and control samples were also evident in a statistical groupwise comparison conducted with edgeR as illustrated in a volcano plot (Fig. 1F). A set of more than 100 miRNAs were detected with a significantly different abundance in tumor samples showing a min. 2-fold difference and a false discovery rate (FDR) < 0.05. Of note, among the most abundant miRNAs were members of the miR-10 family, which were previously shown to be upregulated in RDEB tumor cells and tissue (Wimmer et al. 2020).

### Machine learning-based identification of tumor-associated miRNA patterns.

In order to identify the most relevant miRNA signatures among the numerous possible combinations, a variety of machine learning algorithms differing in their underlying architecture were employed, with the aim to identify combinations of miRNAs that prove most informative for distinguishing tumor from control samples.

The miRNA-seq dataset initially comprising 27 tumor and 34 control samples and over 1000 detected miRNAs was processed as outlined in Fig. 2A. First, low quality samples, defined as such containing no reads mapping to mature miRNAs were dropped (Fig. 2A). In addition, miRNAs with very low read numbers (≤ 5 reads) were dropped to remove noisy signals. A reduced sample set of 26 tumor and 32 control samples with a total of 268 robustly detected miRNAs finally served to train and test the tumor prediction models. The data were used either directly as raw counts or alternatively with prior normalization relative to the three most stable reference miRNAs (SRMs) in combination with min-max scaling to compensate for differences in sequencing depth between the samples.

To this end, differences in miRNA abundance between tumor and control samples were examined with three different standard statistical methods implemented in the Bioconductor packages edgeR, DESeq2 and limma, and the top ranking three miRNAs (miR-29c-5p, miR-625-3p and miR-148b-3p, Fig. 2B) from the intersection of all three methods were selected based on high p-values, indicative for non-significant differences between the conditions (Robinson et al. 2010, Love et al. 2014, Law et al. 2014). Within-sample normalization of miRNAs to the mean of log2-transformed SRMs provided a reasonably balanced dataset compared to raw-data.

Four supervised machine learning algorithms, representing different families of classification strategies, were chosen to provide complementary means of screening the inventors' data for the best tumor predictors. K-top scoring pairs (kTSP), a rule-based, parameter-free classifier, utilizes a set (k) of paired measurements (miRNAs) comparing their abundance with each other (e.g. miR-A > miR-B). This represents a very simple and easy to interpret prediction model not affected by any prior data normalization, owing to its algorithm, which is always considering a feature (miRNA) in relation to another one, thereby conducting an auto-normalization (Afsari et al. 2015).

All other three algorithms depended on SRM normalized, "min-max" scaled data. Extreme gradient boosting (XGBoost) is representative for a decision tree-based method highly efficient in capturing non-linear relations in the data. This method was chosen based on its outstanding ability to minimize bias and variance, which is founded on the "wisdom of the crowd" concept by employing an ensemble of weak learners (small decision trees)(Chen et al. 2016). The initial weak learners are boosted by iterative addition of further weak learners trained on re-weighed data to enforce learning from misclassifications of the predecessors (Chen et al. 2016). Another algorithm used was a support vector machine (SVM), a linear classification model which strives to find a hyperplane in n-dimensional space (miRNAs) separating the samples into classes with a maximal margin between them defined by the data points - support vectors (miRNA abundance) closest to the hyperplane (Huang et al. 2017). Finally, glmnet, a classifier based on logistic regression was applied, which offers an integrated regularization to minimize overfitting effects (Zou et al. 2005).

Model training was conducted by grid search and repeated (n = 30) 10-fold cross-validation to optimize model parameters and provide corresponding prediction performance metrics following standard procedures offered by caret's R package (Kuhn et al. 2008). First, the learning algorithms were allowed to use all 268 miRNAs as features for their prediction models, which is referred herein as "full" models. In order to restrain the risk of overfitting the inventors performed a second round of training to provide "sparse" models, with the exception of the kTSP algorithm, which automatically searched for a parsimonious model choosing a set of two miRNA pairs combining three miRNAs (miR-1260a, miR-301a-3p, miR-187-3p) without requiring further optimization.

For the second training round, only a limited set of miRNAs was provided during model training, based on a feature importance analysis derived from the initial round. Inspection of a feature importance plot of miRNAs used by the "full" XGBoost model highlighted the distinct relevance of two miRNAs (miR-27b-3p and miR-187-3p), which were subsequently selected for the training of a "sparse" model. No such clear trend in feature importance was observed for SVM and glment.

To use about the same number of features as in the other models and thereby allow a similar model complexity, the inventors opted to limit the features to the three top and thus most important miRNAs (glmnet: miR-769-3p, miR-22-5p, miR125a-3p; SVM: miR-198-3p, miR-95-3p, miR-193b-3p). For the performance evaluation of the different prediction models the inventors chose to use the area under the receiver operating characteristic curve (AUROC), a metric which is not biased by thresholds and prevalence (Wang et al. 2021). A comparison of the discrimination accuracy (Fig. 2C, Tab. 2) between all four tested models ranked kTSP and XGBoost as top performers, with both full and sparse model complexity.

**Table 2: Performance comparison**

| Area under the receiver operating characteristic curve (AUROC) of all four tested models derived from repeated (n = 30) 10-fold cross-validation out of the complete pool of control (n = 32) and tumor (n = 24) samples. Number (#) of features used by each model, where "full" refers to allowing the algorithm to pick features from either all detected miRNAs (n = 268) or limiting the choice to the most important features used by "sparse" models. AUROC data is presented as mean ± s.e.m. of all 30 repeats x 10-fold performance evaluations. | | | | |
|---|---|---|---|---|
| model | full model | full model | sparse model | sparse model |
| | AUROC | # of | AUROC | # of |
| | (%) | features | (%) | features |
| kTSP | 93.4 ± 1.3 | 3 | 93.4 ± 1.3 | 3 |
| XGBoost | 89.0 ± 0.9 | 268 | 92.3 ± 0.7 | 2 |
| SVM | 84.2 ± 2.1 | 268 | 84.2 ± 2.3 | 3 |
| glmnet | 83.9 ± 2.1 | 268 | 79.1 ± 2.3 | 3 |

Closer examination of the predictors of these two models confirmed significantly (p < 0.005) different miRNA levels in tumor lesion swab specimens (Fig. 3). Based on the inventors' results the inventors conclude that the proposed swab-based sampling method enables retrieval of molecular information sufficient to model tumor prediction algorithms, which in turn demonstrated a promising performance in discriminating tumor lesions from non-cancerous wounds.

### Case study demonstrates the ability of prediction models to accurately distinguish several local tumor spots within a large chronic wound

RDEB patients are burdened with extensive, long-standing chronic wounds, which require close monitoring as tumors tend to arise in those areas. To test the accuracy of the inventors' tumor prediction models in differentiating tumor lesions within a chronic wound area, the inventors used the opportunity of a case study of a 37-year old RDEB patient with a history of multiple, recurrent SCCs within a large chronic wound covering about 80% of her left arm. The patient had undergone multiple surgeries as well as several courses of electrochemotherapy (Bleomycin) due to recurrent, incompletely resected and newly evolving tumors at this site. Subsequent to a follow-up PET-CT scan revealing further scattered residual cancer nodules, additional electrochemotherapy was scheduled and, upon patient's informed consent, provided us with the chance for a profound sampling. In total, the inventors collected swabs as well as site-matched curette biopsies from 23 different sites within the wound area (Fig. 4A-B). Sampling sites were selected in consultation with the responsible clinician and represented a mix of visually suspicious and unsuspicious areas. Sites were finally annotated as tumor or control based on subsequent clinical histopathology reports. A voting scheme (Fig. 4C) summarizes the results of each pairwise signature miRNA comparisons performed by the kTSP model which are combined into a majority vote resulting in the final call for either tumor or control for each swab sample. XGBoost provides a probability of a sample being a tumor given the abundance of its signature miRNAs (Fig. 4D). Both models were able to predict tumor samples with high accuracy (kTSP: 91.3%, XGBoost: 100%, Fig. 4E-F), outcompeting the ability of four trained dermatologists to label suspicious areas correctly (Fig. 4G-J) based on visual inspection of photographs of the wound with one rater knowing the patient and the respective wound, while the other three did not (Fig. 4A). Notably, visual assessment and categorization based on two-dimensional images lacks information that supports clinical decision making, including three-dimensional cues, clinical data on the (patient-specific) course of the lesions, or accompanying symptoms.

Identification of evolving tumor lesions at an early stage within chronic EB wounds remains highly challenging, even for the EB-experienced, and underscores the need for less-invasive approaches to increase diagnostic accuracy to support clinicians in taking care of their patients. This case study presents a realistic clinical scenario and illustrates the advantage of a swab-based sampling method to interrogate suspicious lesions to an extent which would not be justifiable with traditional invasive methods on a routine basis.

### Translation of prediction models to qPCR-based assay

The inventors' sequencing approach provided unbiased insights into the miRNA profiles of wound and tumor swabs. Despite the fact that sequencing is becoming more affordable, the costs and also hands-on time spent preparing the sequencing library are still limiting its use in a diagnostic assay. Ever since the recent Coronavirus pandemic, qPCR has become even more readily available to labs and as services to clinical hospitals. Therefore, the inventors considered a transfer of the inventors' sequence-based findings to a more cost-effective qPCR system with short turnaround times.

To allow seamless transfer of predictive models originally trained on miRNA-seq data to a semi-qPCR assay, the inventors decided in advance to apply a traditional normalization strategy from relative quantification in semi-qPCR experiments to compensate for differences in sequencing depth in the inventors' data (Livak et al. 2001).

Consequently, based on the inventors' prior analysis of the sequencing data (Fig. 2B), the inventors chose to use the most stable SRM, miR-29c-5p, in combination with 5S-rRNA, a short ribosomal RNA commonly used in miRNA qPCR assays as references to determine ΔCq values of the inventors' signature miRNAs. In addition to the necessity of an appropriate reference strategy, there was also the challenge that each assay might entail specific biases that might hinder a one-to-one translation of the inventors' miRNA-based predictions models to qPCR assays (Hong et al. 2021). Therefore, the inventors first tested if ΔCq values of the four signature miRNAs correlated with log2 transformed, TMM normalized sequencing counts (Fig. 5A). All four signature miRNAs and the reference miRNA were detectable in the qPCR assay and three out of the four signature miRNAs demonstrated significant (p < 0.02) correlation between sequencing counts and qPCR ΔCq values. Because miR-1260a lacks coherent abundance between the two assays and thus affects one of two voting rules of the kTSP model, the inventors relaxed the majority vote rule so that one of two votes was sufficient to predict a tumor sample. The prediction accuracy of the kTSP model was slightly higher (80.9%) compared to XGBoost (75.2%) (Fig. 5B, C). Plotting the prediction accuracy over the range of possible threshold values suggested that shifting the cutoff from default 50% to 96% tumor probability would further increase prediction accuracy of XGBoost model (Fig. 5D). In addition to adapting the threshold of XGBoost, the inventors also considered combining outcomes of both models following a trend in machine learning to use ensembles of classifiers, which were frequently shown to gain superior accuracy (Opitz et al. 1999). When aggregating the votes of the kTSP and XGBoost predictions, the accuracy of the qPCR-based assays could be boosted up to 85% as the individual models act complementary in finding true positives (Fig. 5E).

Overall, this demonstrated the feasibility of transferring the prediction models, initially trained on miRNA-seq data, to a qPCR platform with only moderate losses in tumor predictions performance and just required the adjustment of threshold parameter settings.

In addition, to provide an outlook on how a possible diagnostic test could be implemented in practice, the inventors also specified the procedure and required materials necessary to perform the assay as outlined in Fig. 5F. Although routine labs would typically have all required equipment available, a lack of access to bioinformatic resources to interpret the data and run the prediction models might pose a limitation. Therefore, the inventors also developed a Shiny app to demonstrate that the analysis in principle can be carried out on any standard browser without technical specialists.

### References

Afsari B, Fertig EJ, Geman D, Marchionni L. switchBox: an R package for k-Top Scoring Pairs classifier development. Bioinformatics. 2015 Jan 15;31(2):273-4. doi: 10.1093/bioinformatics/btu622. Epub 2014 Sep 26. PMID: 25262153; PMCID: PMC4287945.
Aparicio-Puerta E, Lebrón R, Rueda A, Gómez-Martín C, Giannoukakos S, Jaspez D, et al. sRNAbench and sRNAtoolbox 2019: intuitive fast small RNA profiling and differential expression. Nucleic Acids Res. Oxford Academic; 2019;47:W530-5
Bartel DP. MicroRNAs: genomics, biogenesis, mechanism, and function. Cell. 2004 Jan 23;116(2):281-97. doi: 10.1016/s0092-8674(04)00045-5. PMID: 14744438.
Boeri M, Pastorino U, Sozzi G. Role of microRNAs in lung cancer: microRNA signatures in cancer prognosis. Cancer J. 2012 May-Jun;18(3):268-74. doi: 10.1097/PPO.0b013e318258b743. PMID: 22647364.
Bruce EA, et al. Direct RT-qPCR detection of SARS-CoV-2 RNA from patient nasopharyngeal swabs without an RNA extraction step. PLoS Biol. 2020 Oct 2;18(10):e3000896. doi: 10.1371/journal.pbio.3000896. PMID: 33006983; PMCID: PMC7556528.
Calin GA, Croce CM. MicroRNA signatures in human cancers. Nat Rev Cancer. 2006 Nov;6(11):857-66. doi: 10.1038/nrc1997. PMID: 17060945.
G. A. Calin et al., `Frequent deletions and down-regulation of micro- RNA genes miR15 and miR16 at 13q14 in chronic lymphocytic leukemia', PNAS, vol. 99, no. 24, pp. 15524-15529, Nov. 2002, doi: 10.1073/pnas.242606799
Chacón-Solano E, et al. Fibroblast activation and abnormal extracellular matrix remodelling as common hallmarks in three cancer-prone genodermatoses. Br J Dermatol. 2019 Sep;181(3):512-522. doi: 10.1111/bjd.17698. Epub 2019 Apr 15. PMID: 30693469; PMCID: PMC6850467.
Chen Tianqi, Guestrin Carlos, XGBoost: A Scalable Tree Boosting System KDD '16: Proceedings of the 22nd ACM SIGKDD International Conference on Knowledge Discovery and Data MiningAugust 2016 Pages 785-794, https://doi.org/10.1145/2939672.2939785
Cho RJ, et al. APOBEC mutation drives early-onset squamous cell carcinomas in recessive dystrophic epidermolysis bullosa. Sci Transl Med. 2018 Aug 22;10(455):eaas9668. doi: 10.1126/scitranslmed.aas9668. PMID: 30135250.
Cianfarani F, Zambruno G, Castiglia D, Odorisio T. Pathomechanisms of Altered Wound Healing in Recessive Dystrophic Epidermolysis Bullosa. Am J Pathol. 2017 Jul; 187(7): 1445-1453. doi: 10.1016/j.ajpath.2017.03.003. Epub 2017 Apr 29. PMID: 28460207.
Condorelli AG, Dellambra E, Logli E, Zambruno G, Castiglia D. Epidermolysis Bullosa-Associated Squamous Cell Carcinoma: From Pathogenesis to Therapeutic Perspectives. Int J Mol Sci. 2019;20(22):5707. Published 2019 Nov 14. doi:10.3390/ijms20225707
M. A. Cortez, et al. 'MicroRNAs in body fluids-the mix of hormones and biomarkers', Nat Rev Clin Oncol, vol. 8, no. 8, pp. 467-477, Jun. 2011, doi: 10.1038/nrclinonc.2011.76.
Croce CM. Causes and consequences of microRNA dysregulation in cancer. Nat Rev Genet. 2009 Oct;10(10):704-14. doi: 10.1038/nrg2634. PMID: 19763153; PMCID: PMC3467096. Cui M, Wang H, Yao X, Zhang D, Xie Y, Cui R, Zhang X. Circulating MicroRNAs in Cancer: Potential and Challenge. Front Genet. 2019 Jul 18;10:626. doi: 10.3389/fgene.2019.00626. PMID: 31379918; PMCID: PMC6656856.
Ding L, et al. Promising therapeutic role of miR-27b in tumor. Tumour Biol. 2017 Mar;39(3): 1010428317691657. doi: 10.1177/1010428317691657. PMID: 28351320.
Fang H, et al. Proinflammatory role of blister fluid-derived exosomes in bullous pemphigoid. J Pathol. 2018 May;245(1): 114-125. doi: 10.1002/path.5061. Epub 2018 Apr 2. PMID: 29468680.
Fine JD, Johnson LB, Weiner M, Li KP, Suchindran C. Epidermolysis bullosa and the risk of life-threatening cancers: the National EB Registry experience, 1986-2006. J Am Acad Dermatol. 2009 Feb;60(2):203-11. doi: 10.1016/j.jaad.2008.09.035. Epub 2008 Nov 20. PMID: 19026465.
Fuentes I, et al. Reduced Microbial Diversity Is a Feature of Recessive Dystrophic Epidermolysis Bullosa-Involved Skin and Wounds. J Invest Dermatol. 2018 Nov; 138(11):2492-2495. doi: 10.1016/j.jid.2018.04.026. Epub 2018 May 16. PMID: 29753707.
Farshchian, Mehdi et al. "Serpin peptidase inhibitor clade A member 1 (SerpinA1) is a novel biomarker for progression of cutaneous squamous cell carcinoma." The American journal of pathology vol. 179,3 (2011): 1110-9. doi:10.1016/j.ajpath.2011.05.012
Fortunato O, et al. Circulating mir-320a promotes immunosuppressive macrophages M2 phenotype associated with lung cancer risk. Int J Cancer. 2019 Jun 1; 144(11):2746-2761. doi: 10.1002/ijc.31988. Epub 2019 Jan 6. PMID: 30426475; PMCID: PMC6590261.
Ghafour AA, et al. High expression level of miR-1260 family in the peripheral blood of patients with ovarian carcinoma. J Ovarian Res. 2021 Oct 10;14(1):131. doi: 10.1186/s13048-021-00878-x. PMID: 34629107; PMCID: PMC8504092.
Gruber C, et al. The design and optimization of RNA trans-splicing molecules for skin cancer therapy. Mol Oncol. 2013 Dec;7(6):1056-68. doi: 10.1016/j.molonc.2013.08.005. Epub 2013 Aug 19. PMID: 23998959; PMCID: PMC5528447.
Guttmann-Gruber, C., et al., Low-dose calcipotriol can elicit wound closure, anti-microbial, and anti-neoplastic effects in epidermolysis bullosa keratinocytes. Sci Rep, 2018. 8(1): p. 13430.
Guo J, et al. miR-301a-3p induced by endoplasmic reticulum stress mediates the occurrence and transmission of trastuzumab resistance in HER2-positive gastric cancer. Cell Death Dis. 2021 Jul 13;12(7):696. doi: 10.1038/s41419-021-03991-3. PMID: 34257270; PMCID: PMC8277821.
Hanahan D, Weinberg RA. Hallmarks of cancer: the next generation. Cell. 2011 Mar 4;144(5):646-74. doi: 10.1016/j.cell.2011.02.013. PMID: 21376230.
Has C, et al. Consensus reclassification of inherited epidermolysis bullosa and other disorders with skin fragility. Br J Dermatol. 2020 Oct;183(4):614-627. doi: 10.1111/bjd.18921. Epub 2020 Mar 11. PMID: 32017015.
Hong LZ, et al. Systematic evaluation of multiple qPCR platforms, NanoString and miRNA-Seq for microRNA biomarker discovery in human biofluids. Sci Rep. 2021 Feb 24;11(1):4435. doi: 10.1038/s41598-021-83365-z. PMID: 33627690; PMCID: PMC7904811.
Huang MW, Chen CW, Lin WC, Ke SW, Tsai CF. SVM and SVM Ensembles in Breast Cancer Prediction. PLoS One. 2017;12(1):e0161501. Published 2017 Jan 6. doi: 10.1371/journal.pone.0161501
Kim M, Li M, Intong-Wheeler LRA, Tran K, Marucci D, Murrell DF. Epidemiology and Outcome of Squamous Cell Carcinoma in Epidermolysis Bullosa in Australia and New Zealand. Acta Derm Venereol. 2018 Jan 12;98(1):70-76. doi: 10.2340/00015555-2781. PMID: 28853495.
Kivisaari AK, et al. Transformation-specific matrix metalloproteinases (MMP)-7 and MMP-13 are expressed by tumour cells in epidermolysis bullosa-associated squamous cell carcinomas. Br J Dermatol. 2008 Apr;158(4):778-85. doi: 10.1111/j.1365-2133.2008.08466.x. Epub 2008 Feb 16. PMID: 18284387.
Kuhn, M. (2008). Building Predictive Models in R Using the caret Package. Journal of Statistical Software, 28(5), 1-26. https://doi.org/10.18637/jss.v028.i05
Law CW, Chen Y, Shi W, Smyth GK. voom: Precision weights unlock linear model analysis tools for RNA-seq read counts. Genome Biol. 2014 Feb 3;15(2):R29. doi: 10.1186/gb-2014-15-2-r29. PMID: 24485249; PMCID: PMC4053721.
Lê S, Josse J, Husson F (2008). "FactoMineR: A Package for Multivariate Analysis." Journal of Statistical Software, 25(1), 1-18. doi: 10.18637/jss.v025.i01.
Lettner, T., et al., Increased levels of matrix metalloproteinase-9 and interleukin-8 in blister fluids of dystrophic and junctional epidermolysis bullosa patients. J Eur Acad Dermatol Venereol, 2015. 29(2): p. 396-8.
LivakKJ, Schmittgen TD. Analysis of relative gene expression data using real-time quantitative PCR and the 2(-Delta Delta C(T)) Method. Methods. 2001 Dec;25(4):402-8. doi: 10.1006/meth.2001.1262. PMID: 11846609.
Love MI, Huber W, Anders S. Moderated estimation of fold change and dispersion for RNA-seq data with DESeq2. Genome Biol. 2014;15(12):550. doi: 10.1186/s13059-014-0550-8. PMID: 25516281; PMCID: PMC4302049.
Mandujano-Tinoco EA, Garcia-Venzor A, Muñoz-Galindo L, Lizarraga-Sanchez F, Favela-Orozco A, Chavez-Gutierrez E, Krötzsch E, Salgado RM, Melendez-Zajgla J, Maldonado V. miRNA expression profile in multicellular breast cancer spheroids. Biochim Biophys Acta Mol Cell Res. 2017 Oct;1864(10):1642-1655. doi: 10.1016/j.bbamcr.2017.05.023. Epub 2017 May 31. PMID: 28576513.
J. Matsuzaki and T. Ochiya, `Circulating microRNAs: Next-generation Cancer Detection', Keio J Med, May 2020, doi: 10.2302/kjm.2019-0011-OA.
Mellerio JE, et al. Management of cutaneous squamous cell carcinoma in patients with epidermolysis bullosa: best clinical practice guidelines. Br J Dermatol. 2016 Jan;174(1):56-67. doi: 10.1111/bjd. 14104. Epub 2015 Nov 7. PMID: 26302137.
Mensah, M., et al. (2017). MicroRNA Based Liquid Biopsy: The Experience of the Plasma miRNA Signature Classifier (MSC) for Lung Cancer Screening. Journal of visualized experiments : JoVE, (128), 56326. https://doi.org/10.3791/56326
P. S. Mitchell et al., `Circulating microRNAs as stable blood-based markers for cancer detection', Proc Natl Acad Sci USA, vol. 105, no. 30, pp. 10513-10518, Jul. 2008, doi: 10.1073/pnas.0804549105
Nyström A, et al. Collagen VII plays a dual role in wound healing. J Clin Invest. 2013 Aug;123(8):3498-509. doi: 10.1172/JCI68127. Epub 2013 Jul 8. PMID: 23867500; PMCID: PMC3726167.
Nyström A, et al. Losartan ameliorates dystrophic epidermolysis bullosa and uncovers new disease mechanisms. EMBO Mol Med. 2015 Sep;7(9): 1211-28. doi: 10.15252/emmm.201505061. PMID: 26194911; PMCID: PMC4568953.
Nyström A, et al. Impaired lymphoid extracellular matrix impedes antibacterial immunity in epidermolysis bullosa. Proc Natl Acad Sci U S A. 2018 Jan 23;115(4):E705-E714. doi: 10.1073/pnas.1709111115. Epub 2018 Jan 5. PMID: 29305555; PMCID: PMC5789908.
Opitz D, Maclin R. Popular Ensemble Methods: An Empirical Study. Journal of Artificial Intelligence Research 11 (1999) 169-198
Pastorino U, et al. Baseline computed tomography screening and blood microRNA predict lung cancer risk and define adequate intervals in the BioMILD trial. Ann Oncol. 2022 Apr;33(4):395-405. doi: 10.1016/j.annonc.2022.01.008. Epub 2022 Jan 25. PMID: 35091076.
Y. Peng and C. M. Croce, `The role of MicroRNAs in human cancer', Signal Transduction and Targeted Therapy, vol. 1, no. 1, Art. no. 1, Jan. 2016, doi: 10.1038/sigtrans.2015.4
Phillips T, et al. Aberrant recruitment of leukocytes defines poor wound healing in patients with recessive dystrophic epidermolysis bullosa. J Dermatol Sci. 2020 Dec;100(3):209-216. doi: 10.1016/j.jdermsci.2020.10.009. Epub 2020 Oct 17. PMID: 33143962.
Pritchard CC, et al. Blood cell origin of circulating microRNAs: a cautionary note for cancer biomarker studies. Cancer Prev Res (Phila). 2012 Mar;5(3):492-497. doi: 10.1158/1940-6207.CAPR-11-0370. Epub 2011 Dec 12. PMID: 22158052; PMCID: PMC4186243.
Riihilä P, et al. Tumour-cell-derived complement components C1r and C1s promote growth of cutaneous squamous cell carcinoma. Br J Dermatol. 2020 Mar;182(3):658-670. doi: 10.1111/bjd.18095. Epub 2019 Jul 28. PMID: 31049937; PMCID: PMC7065064.
R Core Team (2020). R: A language and environment for statistical computing. R Foundation for Statistical Computing, Vienna, Austria. URL https://www.R-project.org/. Robinson MD, Oshlack A. A scaling normalization method for differential expression analysis of RNA-seq data. Genome Biol. 2010;11(3):R25. doi: 10.1186/gb-2010-11-3-r25. Epub 2010 Mar 2. PMID: 20196867; PMCID: PMC2864565.
Rodríguez-Galán A, et al. MiRNA post-transcriptional modification dynamics in T cell activation. iScience. 2021 May 12;24(6):102530. doi: 10.1016/j.isci.2021.102530. PMID: 34142042; PMCID: PMC8188497.
Rossato M, et al. IL-10-induced microRNA-187 negatively regulates TNF-α, IL-6, and IL-12p40 production in TLR4-stimulated monocytes. Proc Natl Acad Sci U S A. 2012 Nov 6;109(45):E3101-10. doi: 10.1073/pnas.1209100109. Epub 2012 Oct 15. PMID: 23071313; PMCID: PMC3494907.
RStudio Team (2020). RStudio: Integrated Development for R. RStudio, PBC, Boston, MA URL http://www.rstudio.com/.
Rupaimoole R, Calin GA, Lopez-Berestein G, Sood AK. miRNA Deregulation in Cancer Cells and the Tumor Microenvironment. Cancer Discov. 2016 Mar;6(3):235-46. doi: 10.1158/2159-8290.CD-15-0893. Epub 2016 Feb 10. PMID: 26865249; PMCID: PMC4783232.
Schmohl, M., et al., Superficial wound swabbing: a novel method of sampling and processing wound fluid for subsequent immunoassay analysis in diabetic foot ulcerations. Diabetes Care, 2012. 35(11): p. 2113-20.
Schwarzenbach H, Nishida N, Calin GA, Pantel K. Clinical relevance of circulating cell-free microRNAs in cancer. Nat Rev Clin Oncol. 2014 Mar;11(3):145-56. doi: 10.1038/nrclinonc.2014.5. Epub 2014 Feb 4. PMID: 24492836.
Shen SJ, Song Y, Ren XY, Xu YL, Zhou YD, Liang ZY, Sun Q. MicroRNA-27b-3p Promotes Tumor Progression and Metastasis by Inhibiting Peroxisome Proliferator-Activated Receptor Gamma in Triple-Negative Breast Cancer. Front Oncol. 2020 Aug 5;10:1371. doi: 10.3389/fonc.2020.01371. PMID: 32850439; PMCID: PMC7419677.
Solis DC, Teng C, Gorell ES, Barriga M, Nazaroff J, Li S, Lu Y, Bruckner A, Marinkovich MP, Tang JY. Classification of 2 distinct wound types in recessive dystrophic epidermolysis bullosa: A retrospective and cohort natural history study. J Am Acad Dermatol. 2021 Nov;85(5):1296-1298. doi: 10.1016/j.jaad.2020.08.118. Epub 2020 Sep 4. PMID: 32896597.
Sozzi G, et al. Clinical utility of a plasma-based miRNA signature classifier within computed tomography lung cancer screening: a correlative MILD trial study. J Clin Oncol. 2014 Mar 10;32(8):768-73. doi: 10.1200/JCO.2013.50.4357. Epub 2014 Jan 13.
Sun Y, et L. Extracellular Vesicles as Biomarkers for the Detection of a Tumor Marker Gene in Epidermolysis Bullosa-Associated Squamous Cell Carcinoma. J Invest Dermatol. 2018 May;138(5):1197-1200. doi: 10.1016/j.jid.2017.11.022. Epub 2017 Dec 2. PMID: 29203361.
Toncic RJ, Petkovic M, Susic SM, Ceovic R, Argenziano G. Use of dermatoscopy in the detection of squamous cell carcinoma in a patient with recessive dystrophic epidermolysis bullosa. Dermatol Pract Concept. 2018;8(3):227-230. Published 2018 Jul 31. doi:10.5826/dpc.0803a15
Torri A, et al. Extracellular MicroRNA Signature of Human Helper T Cell Subsets in Health and Autoimmunity. J Biol Chem. 2017 Feb 17;292(7):2903-2915. doi: 10.1074/jbc.M116.769893. Epub 2017 Jan 11. PMID: 28077577; PMCID: PMC5314185.
Wang J, et al. miR-301a Suppression within Fibroblasts Limits the Progression of Fibrosis through the TSC1/mTOR Pathway. Mol Ther Nucleic Acids. 2020 Sep 4;21:217-228. doi: 10.1016/j.omtn.2020.05.027. Epub 2020 May 26. PMID: 32585629; PMCID: PMC7321782.
L. Wang, M. Han, X. Li, N. Zhang and H. Cheng, "Review of Classification Methods on Unbalanced Data Sets," in IEEE Access, vol. 9, pp. 64606-64628, 2021, doi: 10.1109/ACCES S .2021.3074243.
Wimmer, Monika et al. "A cancer stem cell-like phenotype is associated with miR-10b expression in aggressive squamous cell carcinomas." Cell communication and signaling: CCS vol. 18,1 61. 10 Apr. 2020, doi:10.1186/s12964-020-00550-9
Woodley DT, Cogan J, Mosallaei D, Yim K, Chen M. Characterization of mutant type VII collagens underlying the inversa subtype of recessive dystrophic epidermolysis bullosa. J Dermatol Sci. 2021 Nov;104(2):104-111. doi: 10.1016/j.jdermsci.2021.09.006. Epub 2021 Sep 23. PMID: 34674926; PMCID: PMC8639788.
Yager, D.R., R.A. Kulina, and L.A. Gilman, Wound fluids: a window into the wound environment? Int J Low Extrem Wounds, 2007. 6(4): p. 262-72.
Youn, Y. J., et al. (2021). Neutrophil-derived trail is a proinflammatory subtype of neutrophil-derived extracellular vesicles. Theranostics, 11(6), 2770-2787. https: //doi. org/10.7150/thno.51756.
Zauner R, Wimmer M, Dorfer S, Ablinger M, Koller U, Piñón Hofbauer J, Guttmann-Gruber C, Bauer JW, Wally V. Transcriptome-Guided Drug Repurposing for Aggressive SCCs. Int J Mol Sci. 2022 Jan 17;23(2):1007. doi: 10.3390/ijms23021007. PMID: 35055192; PMCID: PMC8780441.
Zauner R, Wimmer M, Atzmueller S, Proell J, Niklas N, Ablinger M, Reisenberger M, Lettner T, Bauer J W, Wally V. Biomarker discovery in rare malignancies: development of a miRNA signature for RDEB-cSCC. manuscript in preparation for publication in Biomarker Res, 2022.
Zou H, Hastie T. Regularization and variable selection via the elastic net. Journal of the Royal Statistical Society: Series B (Statistical Methodology). 2005;67:301-20.

## Claims

1. A method for diagnosing the risk of a subject to suffer from squamous cell carcinoma (SCC), comprising detecting the expression level of the miR-187-3p molecule, an isomiR, or a precursor thereof in a sample obtained from said subject, wherein a changed expression level of said miRNA, as compared to healthy donors, indicates an increased risk of said subject to suffer from squamous cell carcinoma, and optionally further comprising detecting the expression level of at least one of the miR-27b-3p, miR-301a-3p, or miR-1260a, an isomiR, or a precursor thereof in said sample obtained from said subject, wherein a changed expression level of said at least one miRNA, as compared to healthy donors, further indicates an increased risk of said subject to suffer from squamous cell carcinoma, preferably wherein the sample comprises a liquid biopsy and/or swap sample, such as a wound swab sample, from said subject.

2. A method according to claim 1, wherein a decrease of the expression level of miR-301a-3p and/or miR-1260a when compared to healthy donors indicates an increased risk of said subject to suffer from squamous cell carcinoma, and wherein an increase of the expression level of miR-187-3p and/or miR-27b-3p when compared to healthy donors indicates an increased risk of said subject to suffer from squamous cell carcinoma.

3. The method according to claim 1 or 2, further comprising detecting the expression level of miR-29c-5p as a stable reference miRNA and/or 5S-rRNA as a small reference RNA as a control.

4. The method according to any one of claims 1 to 3, further comprising detecting the expression level of at least one of the miRNA molecules, an isomiR, or a precursor thereof selected from the group consisting of miR-769-3p, miR-22-5p, miR125a-3p, miR-198-3p, miR-95-3p, miR-193b-3p, miR-342-3p, miR-125a-5p2, miR-125a-3p4, miR-941, miR-125a-5p5, miR-10a-5p, let-7e-5p6, miR-34a-5p7, miR-193b-5p8, miR-125a-3p9, miR-1307-5p, miR-34a-5p10, miR-10a-3p, and miR-125a-5p.

5. The method according to any one of claims 1 to 4, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

6. The method according to any one of claims 1 to 5, further comprising the step of diagnosing the subject to suffer from SCC, in particular cutaneous squamous cell carcinoma (cSCC).

7. The method according to any one of claims 1 to 6, wherein the sample from the subject is obtained from said subject either before, during or after a treatment of said subject against wound healing disorders, RDEB and/or SCC, in particular cSCC.

8. A diagnostic composition comprising a sample obtained from a subject at risk to suffer from squamous cell carcinoma (SCC) comprising miR-187-3p molecule, an isomiR, or a precursor thereof, preferably further comprising at least one of miR-27b-3p, miR-301a-3p, or miR-1260a molecule, an isomiR, or a precursor thereof, and more preferably further comprising miR-29c-5p as a stable reference miRNA and/or 5 S-rRNA as a small reference RNA as a control, wherein optionally the sample comprises a liquid biopsy and/or swap sample, such as a wound swab sample, from said subject.

9. The diagnostic composition according to claim 8, further comprising at least one of miRNA molecules, an isomiR, or a precursor thereof selected from the group consisting of miR-769-3p, miR-22-5p, miR125a-3p, miR-198-3p, miR-95-3p, miR-193b-3p, miR-342-3p, miR-125a-5p2, miR-125a-3p4, miR-941, miR-125a-5p5, miR-10a-5p, let-7e-5p6, miR-34a-5p7, miR-193b-5p8, miR-125a-3p9, miR-1307-5p, miR-34a-5p10, miR-10a-3p, and miR-125a-5p.

10. The diagnostic composition according to claim 8 or 9, wherein the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

11. A kit for in vitro assessment of the risk that a subject suffers from squamous cell carcinoma (SCC), comprising buffers and reagents to perform a method according to any one of claims 1 to 7, optionally together with instructions for performing said method, wherein preferably the kit comprises suitable buffers and reagents to perform quantitative polymerase chain reaction (qPCR), and/or for miRNA sequencing, and/or the kit further comprising reagents needed to obtain the exosome preparation of the sample.

12. Use of the kit according to claim 11 for diagnosing a subject to suffer from SCC, in particular cutaneous squamous cell carcinoma (cSCC), in particular according to a method according to any one of claims 1 to 7.

13. A computer program for identifying a subject's risk to suffer from SCC, in particular cutaneous squamous cell carcinoma (cSCC), in particular as determined according to a method according to any one of claims 1 to 7, wherein said program is preferably implemented to run on a computer, wherein preferably the computer program is a Shiny app.

14. A method of preventing or treating squamous cell carcinoma (SCC), in particular cSCC, in a subject, comprising the steps of performing method according to any one of claims 1 to 7, to determine if the subject has an increased probability of suffering or is suffering from squamous cell carcinoma (SCC), in particular cSCC, and selecting an appropriate prevention or therapy for the subject based on the determination, wherein preferably the subject suffers from a dysfunction or loss of the structural extracellular matrix protein collagen type VII, for example caused by mutations within the COL7A1 gene, wound healing disorders, and preferably from a severe recessive subtype of dystrophic epidermolysis bullosa (RDEB).

15. The method according to claim 14, wherein the sample from the subject was obtained from said subject either before, during or after a treatment of said subject against wound healing disorders, RDEB and/or SCC, in particular cSCC.
